(19) **Europäisches Patentamt European Patent Office Office européen des brevets**

(11) **EP 3 770 266 B1**

(12) **EUROPEAN PATENT SPECIFICATION**

(45) Date of publication and mention of the grant of the patent:
**17.06.2026 Bulletin 2026/25**

(21) Application number: **19772115.2**

(22) Date of filing: **01.02.2019**

(51) International Patent Classification (IPC):
*C07K 16/06* (2006.01)  *C12M 1/26* (2006.01)
*C12P 21/02* (2006.01)  *C12N 15/09* (2006.01)
*C07K 16/00* (2006.01)  *C12M 1/00* (2006.01)

(52) Cooperative Patent Classification (CPC):
**C12P 21/02; C07K 16/00; C07K 16/065;**
**C12M 29/04; C12M 29/10; C12M 29/18;**
**C12M 29/26; C12M 33/14; C12M 39/00;**
**C12M 47/02; C12M 47/10;** C07K 2317/14;
C12N 15/09

(86) International application number:
**PCT/JP2019/003689**

(87) International publication number:
**WO 2019/181234 (26.09.2019 Gazette 2019/39)**

(54) **PRODUCT PRODUCTION METHOD**

PRODUKTHERSTELLUNGSVERFAHREN

MÉTHODE DE PRODUCTION DE PRODUIT

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **19.03.2018 JP 2018051261**

(43) Date of publication of application:
**27.01.2021 Bulletin 2021/04**

(73) Proprietor: **FUJIFILM Corporation**
**Tokyo 106-8620 (JP)**

(72) Inventors:
 • **TAKAHASHI, Naoto**
 **Ashigarakami-gun, Kanagawa 258-8577 (JP)**
 • **NAKAI, Shinichi**
 **Ashigarakami-gun, Kanagawa 258-8577 (JP)**

(74) Representative: **Hoffmann Eitle**
**Patent- und Rechtsanwälte PartmbB**
**Arabellastraße 30**
**81925 München (DE)**

(56) References cited:
 WO-A1-2018/159847  WO-A1-2018/159847
 JP-A- 2007 525 984  JP-A- 2016 054 686
 US-A1- 2009 042 253

 • DALM MARCELLA C.F. ET AL: "Effect of feed and bleed rate on hybridoma cells in an acoustic perfusion bioreactor: Part I. Cell density, viability, and cell-cycle distribution", vol. 88, no. 5, 5 December 2004 (2004-12-05), US, pages 547 - 557, XP055798569, ISSN: 0006-3592, Retrieved from the Internet <URL:https://citeseerx.ist.psu.edu/viewdoc/download?doi=10.1.1.652.2527& rep=rep1&type=pdf> DOI: 10.1002/bit.20287
 • SAMANTHA WANG ET AL: "Shear contributions to cell culture performance and product recovery in ATF and TFF perfusion systems", JOURNAL OF BIOTECHNOLOGY, vol. 246, 1 March 2017 (2017-03-01), Amsterdam NL, pages 52 - 60, XP055580748, ISSN: 0168-1656, DOI: 10.1016/ j.jbiotec.2017.01.020

**Description**

BACKGROUND OF THE INVENTION

1. Field of the Invention

The present disclosure relates to a method for producing a product produced by a cell.

2. Description of the Related Art

For production of a biopharmaceutical or the like, a method for collecting a culture product contained in a cell culture solution by filtration is known.

As a technique relating to filtration of a culture product, for example, JP2016-054686A discloses a method for collecting a culture product contained in a culture solution in culture of a cell that produces a culture product, the method including: a step B of feeding a culture solution to a filtration membrane; an alternate flow filtration step C of performing filtration while changing flow of the culture solution so as to reciprocate in a direction parallel to a surface of the filtration membrane, to obtain a permeated liquid; a step D of returning a residual culture solution that remains without permeating through the filtration membrane; and a step G of collecting a culture product from the permeated liquid, in which as the filtration membrane used in the step B, a porous membrane having an average pore diameter of 20 $\mu$m to 100 $\mu$m on a surface on a culture solution side or a porous membrane in which a proportion of a pore having a diameter of less than 20 $\mu$m on a surface on a culture solution side is 50% or less with respect to all the pores of the surface on the culture solution side is used.

Moreover, JP2009-045019A discloses a method for producing a protein by continuous filtration culture in which a culture solution of an animal cell that produces a protein in the culture solution is filtered through a separation membrane, the protein which is a product is collected from a filtrate, an unfiltered solution is retained in the culture solution or refluxed, and a fresh medium is added to the culture solution, in which a porous membrane with a micropore having an average micropore diameter of 0.01 $\mu$m or greater and less than 1 $\mu$m is used as the separation membrane, and a filtration treatment is performed at a transmembrane pressure difference in a range of 0.1 kPa to 20 kPa.

Furthermore, as a method for obtaining a high live cell density in cell culture, JP2007-525984A discloses a method for culturing cells by continuous perfusion culture of a cell culture product containing a cell culture medium and cells, in which the cell culture medium is added to the cell culture product, for example, at a rate of 0.01 to 0.3 nL/cell/day, the cell culture product is circulated over a filter module including hollow fibers, and as a result, outflow of a liquid having a cell density lower than that of the cell culture product occurs and flow in the filter module is alternating tangential flow.

DALM MARCELLA C.F. ET AL ("Effect of feed and bleed rate on hybridoma cells in an acoustic perfusion bioreactor: Part I. Cell density, viability, and cell-cycle distribution", BIOTECHNOLOGY AND BIOENGINEERING , vol. 88, no. 5 5 December 2004) discloses the effect of feed and bleed rate on hybridoma cells in an acoustic perfusion bioreactors.

SAMANTHA WANG ET AL ("Shear contributions to cell culture performance and product recovery in ATF and TFF perfusion systems", JOURNAL OF BIOTECHNOLOGY, vol. 246, 1 March 2017) discloses the shear contributions to cell culture performance and product recovery in ATF and TFF perfusion systems.

WO2018/159847A1 discloses a cell culture apparatus for culturing cell for expressing antibody, has collection flow path that collects components blocked by filter membrane which performs membrane separation process with respect to component.

SUMMARY OF THE INVENTION

In the production of a cell product such as a biopharmaceutical, in order to improve production efficiency of the product, a perfusion culture method, in which a culture solution is continuously filtered and discharged, while a fresh medium containing nutrient components is continuously supplied to a culture tank, has been used.

The perfusion culture method enables cells to be cultured at a high concentration, whereas there is a problem that in a case where a cell concentration is high, a permeation rate of a product through a separation membrane is decreased due to continuous culture and an amount of a collected product is reduced. None of techniques described in JP2016-054686A, JP2009-045019A, and JP2007-525984A address such a problem.

An object to be achieved by an embodiment according to the present disclosure is to provide a product production method in which a product is produced by cell culture and the product is separated from a culture solution using a separation membrane, and which is capable of maintaining a permeation rate of the product through the separation membrane at a high level even in a case of cell culture particularly at a high concentration.

BRIEF DESCRIPTION OF THE DRAWINGS

Fig. 1 is a diagram showing an example of a configuration of a cell culture apparatus applicable to implementation of a product production method according to the present disclosure.

Fig. 2 is a diagram for explaining a filtration area S and a volume Vf of a primary side flow path of a hollow fiber filter.

Fig. 3 is a diagram for explaining a filtration area S of a flat membrane filter.

Fig. 4 is a diagram for explaining a volume Vf of a primary side flow path of the flat membrane filter.

Fig. 5 is a diagram schematically showing an aspect in which antibodies as an example of a product pass through a separation membrane.

Fig. 6 is a diagram for explaining a pore diameter Ds of a sparger and a volume Vg of a single gas generated from the sparger.

Fig. 7 is a graph showing a relationship between the number of days of culture and a number density Nd of fine particles having a particle size of 8 Dp to 30 Dp other than live cells in Examples and Comparative Examples.

Fig. 8 is a graph showing a relationship between the number of days of culture and a bleeding amount in Examples and Comparative Examples.

Fig. 9 is a graph showing a relationship between the number of days of culture and a permeation rate of an antibody through a separation membrane in Examples and Comparative Examples.

Fig. 10 is a graph showing a relationship between a molecular weight cut-off and an average pore diameter of an ultrafiltration membrane.

DESCRIPTION OF THE PREFERRED EMBODIMENTS

Hereinafter, a product production method according to the present disclosure will be described.

In the present disclosure, a range of numerical values described using "to" means a range including the numerical values described before and after "to" as a minimum value and a maximum value respectively.

Regarding the ranges of numerical values gradationally described in the present disclosure, an upper limit value or a lower limit value described in a certain range of numerical values may be substituted with an upper limit value or a lower limit value of another range of numerical values gradationally described. Moreover, regarding the ranges of numerical values described in the present disclosure, an upper limit value or a lower limit value described in a certain range of numerical values may be substituted with the values described in Examples.

In the present disclosure, a combination of two or more preferred aspects is a more preferred aspect.

In the present disclosure, in a case where there is a plurality of kinds of substances corresponding to each component, unless otherwise specified, an amount of each component means a total amount of the plurality of kinds of substances.

In the present disclosure, the term "step" includes not only an independent step, but also a step which cannot be clearly differentiated from other steps as long as the intended purpose of the step is achieved.

In the respective drawings, the constituent elements indicated by the same reference mean the same constituent elements.

The product production method according to the present disclosure includes: a step of culturing a cell which produces a product and is contained in a cell suspension accommodated in a culture vessel; a separation treatment step of extracting the cell suspension from the culture vessel, and separating the cell suspension, using a separation membrane, into a return liquid having a cell concentration higher than that of the cell suspension and a permeated liquid having a cell concentration lower than that of the cell suspension and containing the product by a tangential filtration method; a step of returning the return liquid to the culture vessel; a step of supplying a fresh medium into the culture vessel; and a step of collecting the product, in which in a case where a live cell concentration, which is expressed in a unit of cells/mL, in the cell suspension is denoted by Nc, a pore diameter, which is expressed in a unit of m, of the separation membrane is denoted by Dp, a filtration area, which is expressed in a unit of m$^2$, of the separation membrane is denoted by S, and a volume, which is expressed in a unit of cm$^3$, of a primary side flow path of the separation membrane is denoted by Vf, a number density Nd, which is expressed in a unit of particles/mL, of fine particles having a particle size of 8 Dp to 30 Dp other than live cells in the cell suspension satisfies Expression (1).

$$\mathrm{Nc} \leq \mathrm{Nd} \leq \mathrm{S}/(32 \times \pi \times \mathrm{Vf} \times \mathrm{Dp}^2) \quad \text{Expression (1)}$$

A cell used as the cell which produces a product is not particularly limited, but examples thereof include a eukaryotic cell such as an animal cell, a plant cell, and yeast, a prokaryotic cell such as *Bacillus subtilis,* and *E. coli.* An animal cell such as a CHO cell, a BHK-21 cell, a C127 cell, an NS0 cell, and a SP2/0-Ag14 cell is preferable, and from the viewpoint that analysis has been conducted many times and a genetic engineering technique has been established, a CHO cell is more preferable. Even in a case where a cell does not originally produce a desired product or a production amount is small, for example, by introducing an expression vector, such as a plasmid encoding a protein necessary for producing the product, into the cell, the desired product can be efficiently produced. The product produced by the cell in the present disclosure is not particularly limited as long as the product is a substance produced by the cell in a culture solution, and examples thereof include substances such as alcohol, an enzyme, an antibiotic, a recombinant protein, and an antibody. Among them, the product is preferably a recombinant protein or an antibody and more preferably an antibody.

For example, in a case where the product is an antibody, the antibody may be produced by an animal cell such as a CHO cell. The antibody produced by the animal cell is not particularly limited, but examples thereof include an anti-IL-6 receptor antibody, an anti-IL-6 antibody, an anti-glypican-3 antibody, an anti-CD3 antibody, an anti-CD20 antibody, an anti-GPIIb/IIIa antibody, an anti-TNF antibody, an anti-CD25 antibody, an anti-EGFR antibody, an anti-Her2/neu antibody, an anti-RSV antibody, an anti-CD33 antibody, an anti-CD52 antibody, an anti-IgE antibody, an anti-CD11a antibody, an anti-VEGF antibody, and an anti-VLA4 antibody. The antibody includes not only a monoclonal antibody derived from an animal such as a human being, a mouse, a rat, a hamster, a rabbit, and a monkey but also an artificially modified antibody such as a chimeric antibody, a humanized antibody, and a bispecific antibody.

By culturing the cell which produces a product, a targeted product can be produced. The culture can be performed according to known methods.

As a medium used for culturing the cell producing the product, a liquid medium usually used for culturing cells can be used. For example, an OptiCHO (Life Technologies Corporation, 12681011) medium, a Dulbecco's modified Eagle's medium (DMEM), an Eagle's minimal essential medium (MEM), a RPMI-1640 medium, a RPMI-1641 medium, a F-12K medium, a Ham's F12 medium, an Iscove's modified Dulbecco's medium (IMDM), a McCoy's 5A medium, a Leibovitz's L-15 medium, and a medium such as EX-CELL (trademark) 300 series (JRH Biosciences), CHO-S-SFMII (Invitrogen), CHO-SF (Sigma-Aldrich, Inc.), CD-CHO (Invitrogen), IS CHO-V (FUJIFILM Irvine Scientific.), and PF-ACF-CHO (Sigma-Aldrich, Inc.) can be used.

A serum such as a fetal calf serum (FCS) may be added to the medium. Alternatively, the medium may be a serum-free medium, for example, a complete synthetic medium.

The medium may be supplemented with an additional component such as an amino acid, a salt, saccharides, a vitamin, a hormone, a proliferation factor, a buffer solution, an antibiotic, lipid, a trace element, and a hydrolyzate of a plant protein.

The pH of the medium varies depending on cells to be cultured, but is generally 6.0 to 8.0, preferably 6.8 to 7.6, and more preferably 7.0 to 7.4.

A culture temperature is generally 30°C to 40°C, preferably 32°C to 37°C, and more preferably 36°C to 37°C. The culture temperature may be changed during the culture.

The culture is preferably performed in an atmosphere with a $CO_2$ concentration of 0% to 40% and preferably 2% to 10%.

In the culture, medium exchange, air passing, and/or stirring can be performed as necessary.

The culture of a cell which produces a product can be performed in a culture apparatus (also referred to as a bioreactor) or in another suitable vessel. Examples of the culture apparatus include a fermenter-type tank culture apparatus, an air lift-type culture apparatus, a culture flask-type culture apparatus, a spinner flask-type culture apparatus, a microcarrier-type culture apparatus, a fluidized bed-type culture apparatus, a hollow fiber-type culture apparatus, a roller bottle-type culture apparatus, and a filling tank-type culture apparatus.

As described later, a culture scale is preferably 0.1 L to 5,000 L, more preferably 0.5 L to 3,000 L, and still more preferably 1 L to 2,000 L.

Among steps included in the product production method according to the present disclosure, there are a separation treatment step of extracting the cell suspension from the culture vessel, and separating the cell suspension, using a separation membrane, into a return liquid having a cell concentration higher than that of the cell suspension and a permeated liquid having a cell concentration lower than that of the cell suspension and containing a product by a tangential filtration method; a step of returning the return liquid to the culture vessel; and a step of supplying a fresh medium into the culture vessel.

Since the fresh medium is supplied to supplement at least the culture solution lost as the permeated liquid, it can be said that the product production method according to the present disclosure uses perfusion culture.

The perfusion culture is a culture method in which a fresh medium is added and the used medium is removed at the same time. According to the perfusion culture, it is possible to achieve a high cell concentration which exceeds $1 \times 10^8$ cells/mL. In the typical perfusion culture, beginning with batch culture start-up which lasts for one day or two days, a fresh supplied medium is then continuously, gradationally, and/or intermittently added to a culture product and the used medium is removed at the same time. In the perfusion culture, by using a method such as sedimentation, centrifugal separation, and filtration, the used medium can be removed while maintaining the cell concentration.

An advantage of the perfusion culture is that the culture in which a targeted product is produced is maintained for a longer period of time than a batch culture method or fed-batch culture. Moreover, by selecting a membrane pore diameter of the separation membrane, the culture can be continued while collecting the product outside the system. Therefore, a residence time in the culture solution of the product is shortened, chemical change is reduced, and thus quality of the product can be maintained at a high level.

Culture which is a combination of fed-batch culture or perfusion culture can also be performed. One example of the combination includes culture in which the fed-batch culture accompanied with bolus supply is used to maintain culture of a cell in a proliferation phase, and then the perfusion culture is used to produce a targeted product.

Perfusion may be continuous, gradational, intermittent, or in a form of a combination thereof. Since the pore diameter of the separation membrane is smaller than a cell which produces a product, the cell which produces a product is retained in a cell suspension serving as a culture solution, and a permeated liquid substantially does not contain the cell or contains the cell at a much lower concentration than that of the cell suspension. Since a targeted product expressed by cell culture is smaller than the membrane pore diameter, the product can be transferred to the permeated liquid side and collected.

In the culture of the cell which produces a product, a concentration of seeded cells is generally $0.2 \times 10^6$ cells/mL to $3 \times 10^7$ cells/mL and preferably $0.5 \times 10^6$ cells/mL to $1 \times 10^7$ cells/mL.

In the culture of the cell which produces a product, a percentage of live cells during a culture period is preferably 80% or more, more preferably 85% or more, and still more preferably 90% or more in the entire period. In a case where the percentage of live cells is within the above range, production efficiency of the product is high, and unnecessary substances in the cell suspension can be reduced.

In the culture of the cell which produces a product, a maximum live cell concentration (the highest live cell concentration) is preferably $2 \times 10^8$ cells/mL or lower, more preferably $1.5 \times 10^8$ cells/mL or lower, and still more preferably $1.2 \times 10^8$ cells/mL or lower.

In a case where the maximum live cell concentration is within the above range, a decrease in a permeation rate of the product through the separation membrane can be more effectively suppressed. From the viewpoint of improving the production efficiency of the product, the maximum live cell concentration is preferably $2 \times 10^7$ cells/mL or higher, more preferably $3 \times 10^7$ cells/mL or higher, and still more preferably $4 \times 10^7$ cells/mL or higher.

In the present disclosure, a range defined by freely combining the description of the upper limit value and the description of the lower limit value for each parameter is also within the scope of the matters disclosed in the present disclosure.

In the culture of the cell which produces a product, a perfusion rate is preferably 0.3 vvd to 5.0 vvd and more preferably 0.3 vvd to 3.0 vvd. Here, vvd represents the following.

vvd = (volume of fresh medium/working volume of reactor/day, amount of supplied culture solution/amount of culture solution per day)

The concentration of seeded cells and the maximum cell concentration in the culture can be determined by measuring the number of cells by a conventional method and dividing the number of cells by the amount of the culture solution. The maximum live cell concentration is the maximum value of the live cell concentration during the culture period, and varies depending on culture conditions.

The percentage of live cells (survival rate) during the culture period is determined by dividing the number of live cells by (the number of live cells + the number of dead cells). For example, the number of live cells and the number of dead cells can be measured using Vi-CELL XR (trade name, Beckman Coulter, Inc.) which uses viability determination by a trypan blue staining method.

In the separation treatment step in the product production method according to the present disclosure, the cell suspension is extracted from the culture vessel, and separated, using the separation membrane, into a return liquid having a cell concentration higher than that of the cell suspension and a permeated liquid having a cell concentration lower than that of the cell suspension and containing the product by the tangential filtration method.

The tangential filtration method is a method in which by flowing a liquid to be subjected to the membrane separation treatment along a membrane surface of the separation membrane, components having a small size and liquid components are moved to a permeation side of the separation membrane, and components having a large size and remaining liquid components are retained on a non-permeation side of the separation membrane.

In the present disclosure, the non-permeation side of the separation membrane, that is, the supply side is also referred to as a primary side, and the permeation side of the separation membrane is also referred to as a secondary side.

In the membrane separation treatment by the tangential filtration method in the present disclosure, flow of the cell suspension extracted from the culture vessel in one direction parallel to the membrane surface of the separation membrane may be formed, or reciprocating flow may be formed by reversing the flow direction of the cell suspension extracted from the culture vessel every predetermined time.

In the product production method according to the present disclosure, the term "step" in the step of culturing a cell, the separation treatment step, the step of returning the return liquid to the culture vessel, the step of supplying a fresh medium into the culture vessel, the step of collecting a product, and a bleeding step described later does not mean a treatment which is sequentially performed in a batch, such as starting a next treatment after completion of one treatment, and means a treatment which is performed in parallel in terms of time. Therefore, for example, while performing the culturing step, the cell suspension is extracted from the culture vessel to perform the separation treatment step. Moreover, the feeding of the return liquid to the culture vessel, the supply of the fresh medium into the culture vessel, and the collection of the product are also performed in parallel with the culturing step and the separation treatment step in terms of time. By performing the respective steps in parallel in terms of time, the produced product can be efficiently separated and collected. Furthermore, the respective steps may be continuously performed, but for example, there may be a temporary stop time for switching a flow direction of tangential filtration in the separation treatment step.

The extraction of the cell suspension from the culture vessel can usually be performed by using a pump, but other available liquid feeding units may be used. The cell suspension extracted from the culture vessel is fed to a filter part. The extraction of the cell suspension from the culture vessel to the filter part may be performed intermittently or continuously, but is preferably performed continuously from the viewpoint that a system state is stably maintained.

The filter part comprises, for example, a vessel, and a separation membrane which separates a space inside the vessel into a supply side and a permeation side and performs a membrane separation treatment on the cell suspension extracted from the culture vessel. On the supply side of the separation membrane, the filter part has an inlet port and an outlet port, each of which is connected to a pipe. In the membrane separation treatment by the tangential filtration method, in a case where the flow direction of the cell suspension extracted from the culture vessel is reversed every predetermined time, the inlet port and the outlet port are switched by reversing the flow direction.

As the separation membrane, a mesh filter formed by weaving fibrous members in a mesh shape can be used. By using the mesh filter, discharge of components unnecessary for cell culture, which include dead cells and crushed cells, to a permeation side can be promoted, compared to a case where a hollow fiber membrane is used. Accordingly, components unnecessary for cell culture can be effectively removed from the culture vessel, and proliferative properties of cells in the culture vessel can be improved.

In addition, as the separation membrane, a hollow fiber membrane can be used. By using the hollow fiber membrane, a risk of permeation of cells to a permeation side can be reduced, compared to a case where a mesh filter is used. Moreover, a risk of occurrence of clogging due to entry of cells into the separation membrane can be reduced. Accordingly, loss of cells can be reduced. In this case, as the hollow fiber membrane, a microfiltration membrane (MF membrane) or an ultrafiltration membrane (UF membrane) can be used.

The separation membrane does not allow live cells in the cell suspension to permeate, but allows a targeted product in the cell suspension to permeate. The reason for a difference in permeability between the live cell and the product is that there is a difference between the size of the live cell and the size of the targeted product. For example, in a case where an antibody is produced by an animal cell, a size of the antibody is about 1 nm to 5 nm whereas a size of the animal cell is about 10 $\mu$m order, and thus, for example, by using a microfiltration membrane or an ultrafiltration membrane as the separation membrane, the antibody can be transferred to the secondary side through the separation membrane while remaining the live cell on the primary side. Moreover, since sizes of liquid components, for example, water molecules are minute, some liquid components are also transferred together with the antibody to the secondary side. As a result, the cell concentration in the liquid remaining on the primary side is increased. As described above, by performing the separation by the tangential filtration method, the cell suspension fed to the filter part is separated into a return liquid which remains on the primary side without permeating the separation membrane and has a cell concentration higher than that of the cell suspension and a permeated liquid which permeates the separation membrane to be transferred to the secondary side and has a cell concentration lower than that of the cell suspension. The permeated liquid contains a targeted product.

Furthermore, the cell concentration in the cell suspension mentioned herein means a concentration of live cells in the cell suspension in the culture vessel.

The "cell concentration" in the present disclosure refers to a "live cell concentration", unless otherwise specified.

The return liquid remaining on the primary side flows out from the filter part and is returned to the culture vessel.

For example, in a case where a flow direction in a space on the supply side of the filter part in the tangential filtration is fixed to one direction, the cell suspension may be allowed to flow out from the outlet port by liquid feeding pressure through the same pump as that for the cell suspension supplied from the culture vessel to the filter part, and may be returned to the culture vessel through a pipe which connects the outlet port and the culture vessel. In this case, circulating flow is formed by returning the return liquid to the culture vessel through a pipe different from a pipe through which the cell suspension in the culture vessel is moved to the inlet port of the filter part.

On the other hand, in a case where a flow direction in a space on the supply side of the filter part in the tangential filtration is reversed with the lapse of time, that is, the flow is reciprocated, for example, the flow may be reciprocated according to a moving direction of a diaphragm using a diaphragm pump or the like which is used as both a suction port and a jetting port. In this case, the return liquid is returned to the culture vessel through the same pipe as a pipe through which the cell suspension in the culture vessel is moved to the inlet port of the filter part, and with reversal of the flow direction, the inlet port and the outlet port are reversed.

In a case where the circulating flow is formed, for example, KrosFlo perfusion culture flow path device (KML-100, KPS-200, or KPS-600) of Spectrum Laboratories Inc. or PuraLev series manufactured by Levitronix can be suitably used. Moreover, in a case where the reciprocating flow is formed, an ATF system of Repligen Corporation can be suitably used.

A targeted product is contained in the permeated liquid permeating the separation membrane. The product in the permeated liquid is collected, and can be used for various applications such as production of pharmaceuticals and production of food.

The collection of the product may be simply recovery of the permeated liquid, for example, recovery of permeated liquid in a tank. In a case where purity of the product is desired to be improved, a solvent is desired to be changed, or a form is desired to be changed, for example, into a powder form, the permeated liquid can be subjected to an additional treatment.

For example, the product which is contained in the permeated liquid permeating the separation membrane can be purified by a purification treatment. The obtained product can be purified to high purity.

In a case where the product is a polypeptide such as an antibody or a fragment thereof, separation and purification of the product may be performed by using the separation and purification method usually used for polypeptides. The polypeptide can be separated and purified by appropriately selecting and combining, for example, a chromatography column such as affinity chromatography, a filter, ultrafiltration, salting out, dialysis, SDS polyacrylamide gel electrophoresis, isoelectric focusing electrophoresis, and the like, but the method is not limited thereto. A concentration of the obtained polypeptide can be measured by measurement of absorbance, enzyme-linked immunosorbent assay (ELISA), or the like.

Examples of the column used for the affinity chromatography include a protein A column and a protein G column. Examples of chromatography other than the affinity chromatography include ion exchange chromatography, hydrophobic chromatography, gel filtration, reverse phase chromatography, and adsorption chromatography. The chromatography can be performed using liquid phase chromatography such as high performance liquid chromatography (HPLC) and fast protein liquid chromatography (FPLC).

Since the cell suspension fed from the culture vessel to the filter part is separated into the permeated liquid and the return liquid, an amount of the return liquid is smaller than an amount of the cell suspension fed to the filter part.

In order to supplement at least a difference between the amount of the cell suspension fed to the filter part and the amount of the return liquid, a fresh medium is supplied into the culture vessel. By supplying the fresh medium into the culture vessel, it is possible to suppress an excessive increase in the cell concentration in the culture vessel, and to maintain a state of the cell in the culture vessel in a healthy state. That is, advantages of the perfusion culture are obtained.

In a case where there is the cell suspension lost from the culture vessel in addition to the liquid fed to the filter part due to an extraction operation in the bleeding step described later, the supply of the fresh medium into the culture vessel is performed to also supplement the amount of the cell suspension lost due to an extraction operation in the bleeding step, that is, to maintain the amount of the cell suspension in the culture vessel substantially constant. That is, in a case where the bleeding step is performed, the supply of the fresh medium into the culture vessel also includes a fresh medium addition operation in the bleeding step. It can also be said that the addition of the fresh medium into the culture vessel in the bleeding step is performed as a part of the supply of the fresh medium into the culture vessel.

The addition of the fresh medium to supplement the amount of the liquid lost as the permeated liquid and the addition of the fresh medium to supplement the liquid amount of the cell suspension extracted in the bleeding step may be performed through the same pipe or may be performed through separate pipes.

Furthermore, the supply of the fresh medium into the culture vessel may be performed intermittently or continuously. Moreover, the amount of the cell suspension in the culture vessel is not necessary to be completely constant at all times, and may be a substantially constant amount, for example, with a variation of about $\pm$ 10%, $\pm$ 5%, or $\pm$ 1%.

The measurement of the amount of the cell suspension in the culture vessel is not necessary to be performed at all times, and may be performed intermittently.

Even with a slight variation in the amount of the cell suspension, the advantages of the perfusion culture can be obtained.

In the product production method according to the present disclosure, in a case where a live cell concentration, which is expressed in a unit of cells/mL, in the cell suspension is denoted by Nc, a pore diameter, which is expressed in a unit of m, of the separation membrane is denoted by Dp, a filtration area, which is expressed in a unit of $m^2$, of the separation membrane is denoted by S, and a volume, which is expressed in a unit of $cm^3$, of a primary side flow path of the separation membrane is denoted by Vf, a number density Nd, which is expressed in a unit of particles/mL, of fine particles having a particle size of 8 Dp to 30 Dp other than live cells in the cell suspension satisfies Expression (1).

$$Nc \leq Nd \leq S/(32 \times \pi \times Vf \times Dp^2) \quad \text{Expression (1)}$$

The live cell concentration Nc in the cell suspension can be determined by using Cell Viability Analyzer Vi-CELL XR (trade name) of Beckman Coulter, Inc.

In a case where the separation membrane is a microfiltration membrane or an ultrafiltration membrane, the pore diameter Dp of the separation membrane can be measured as an average pore diameter by a mercury press-in method. For example, the pore diameter Dp can be measured by pressing mercury into the separation membrane with high pressure by using AUTOPORE IV 9520 manufactured by Shimadzu Corporation.

Furthermore, the ultrafiltration membrane (UF membrane: also referred to as an Ultrafiltration Membrane in some cases) is a filtration membrane that has an average pore diameter of 0.001 μm to 0.01 μm, which is a smaller average pore diameter than that of the microfiltration membrane (MF membrane: also referred to as a Microfiltration Membrane in some cases), and in which a separation performance is defined by a molecular weight cut-off.

In the ultrafiltration membrane having a small pore diameter, the measurement of the average pore diameter by a mercury press-in method may be difficult. In this case, the average pore diameter of the ultrafiltration membrane can be estimated based on the molecular weight cut-off. Specifically, as described in http://chemeng.in.coocan.jp/memb/m_mb4. html, a molecular weight of a standard substance, which is obtained in a case where a blocking rate is 90% after permeation of a plurality of kinds of standard substances having known molecular weights through a target ultrafiltration membrane, is defined as a molecular weight cut-off of this ultrafiltration membrane.

The blocking rate is a value provided by 1 - (Cp/Cb) in a case where a concentration of a target substance in a solution before filtration is denoted by Cb, and a concentration of a target substance in the filtrate (permeated liquid) is denoted by Cp.

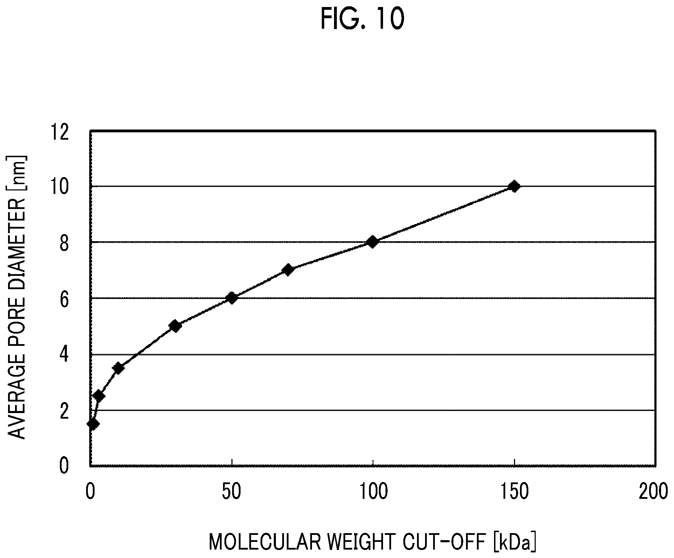

Since a molecular size can be estimated from a molecular weight of a standard substance, the average pore diameter of the ultrafiltration membrane can be estimated based on a molecular weight cut-off. Specifically, as described in Table 1, an average pore diameter is estimated from a molecular weight cut-off. In a case where a molecular weight cut-off does not match a molecular weight of a standard substance, an average pore diameter can be estimated by plotting a molecular weight cut-off and an average pore diameter and performing interpolation by linear interpolation or the like. As an example, Fig. 10 shows an example in a case where the molecular weight cut-off of Table 1 is plotted and interpolated. The estimated values obtained in this manner can be used as the average pore diameter of the ultrafiltration membrane.

From the viewpoint that the product is allowed to efficiently permeate while suppressing permeation of the cell which produces a product, the pore diameter Dp of the separation membrane is preferably 0.1 μm to 1 μm, more preferably 0.15 μm to 0.8 μm, still more preferably 0.17 μm to 0.23 μm, and most preferably 0.2 μm.

A number density of fine particles having a particle size of 8 Dp to 30 Dp can be measured using Multisizer 4e (trade name) of Beckman Coulter, Inc. That is, the particle size in the present disclosure is a particle size determined based on the Coulter principle by using Multisizer 4e (trade name) of Beckman Coulter, Inc. The number density Nd of fine particles having a particle size of 8 Dp to 30 Dp other than live cells can be determined by subtracting the concentration of the live cells having a particle size of 8 Dp to 30 Dp from the number density of the fine particles having a particle size of 8 Dp to 30 Dp.

[Table 1]

| Molecular weight cut-off (kDa) | Estimated value of average pore diameter (nm) |
| --- | --- |
| 1 | 1.5 |
| 3 | 2.5 |
| 10 | 3.5 |
| 30 | 5 |
| 50 | 6 |
| 70 | 7 |
| 100 | 8 |
| 150 | 10 |

The filtration area S is a membrane area on the primary side of the separation membrane.

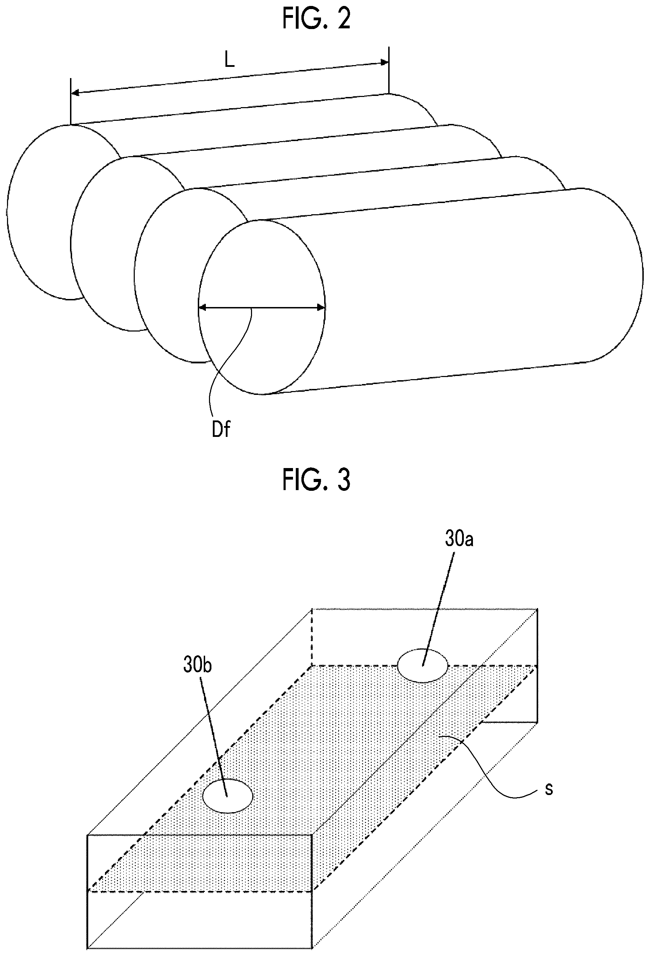

For example, in a case where the separation membrane is a hollow fiber membrane having a shape as shown in Fig. 2, a filtration area per one membrane is determined as $\pi \times Df \times L$ based on a diameter Df and a length L. In a case where there are N hollow fiber membranes in total, the entire filtration area S is determined as $S = \pi \times Df \times L \times N$.

Alternatively, in a case where the separation membrane is a flat membrane having a shape as shown in Fig. 3, an upper side is a primary side, and a lower side is a secondary side, the filtration area S is determined as an area indicated by a stippled portion in Fig. 3. Moreover, in Fig. 3, an inlet port 30a and an outlet port 30b toward a filter part including the separation membrane are provided on the primary side.

The volume Vf of the primary side flow path of the separation membrane is a volume of a space existing in contact with the separation membrane in a space inside the filter part, and can be also said to be a volume of a space existing on the primary side in a direction perpendicular to the membrane surface of the separation membrane in the space inside the filter part.

For example, in a case where the separation membrane is a hollow fiber membrane having a shape as shown in Fig. 2, the inside of the hollow fiber membrane is a primary side, and there are N hollow fiber membranes in total, the volume Vf of the primary side flow path of the separation membrane is determined as $\pi \times (Df^2/4) \times L \times N$ based on the diameter Df and the length L.

On the contrary, in a case where the outside of the hollow fiber membrane is a primary side and there are N hollow fiber membranes in total, the volume Vf of the primary side flow path of the separation membrane is a volume of a space between a vessel (not shown) housing the hollow fiber membrane and the hollow fiber membrane, and is determined by subtracting $\pi \times (Df^2/4) \times L \times N$ from an inner volume of the vessel housing the hollow fiber membrane.

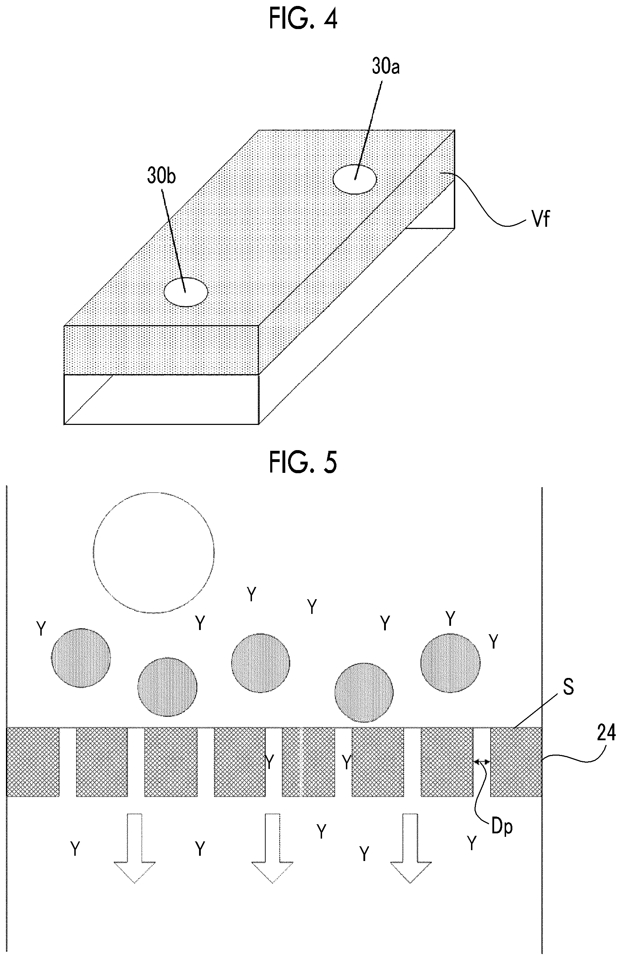

Alternatively, in a case where the separation membrane is a flat membrane having a shape as shown in Fig. 4, an upper side is a primary side, and a lower side is a secondary side, the volume Vf of the primary side flow path of the separation membrane is determined as a volume indicated by a stippled portion in Fig. 4.

In the product production method according to the present disclosure, a number density Nd of fine particles (hereinafter, also referred to as "specific fine particles") which have a particle size of 8 Dp to 30 Dp in the cell suspension and are other than live cells is controlled to be within a range represented by Expression (1).

The reason why the product production method according to the present disclosure is capable of maintaining the permeation rate of the product through the separation membrane at a high level even in a case where cells are cultured at a high concentration is not completely clear, but is presumed to be as follows.

By setting Nd to be equal to or higher than the live cell concentration Nc in the cell suspension, it is possible to effectively obtain an effect of mitigating damage to cells caused by colliding with or getting into a membrane wall of the separation membrane. That is, it is effective that the specific fine particles are present in a certain amount to mitigate damage to cells. Therefore, the product production method according to the present disclosure is significantly different from the technique aimed at simply discharging fine particles in the cell suspension by treating the fine particles as waste products.

In addition, by setting Nd to be equal to or lower than $S/(32 \times \pi \times Vf \times Dp^2)$, it is possible to effectively obtain an effect of suppressing a decrease in a permeation rate (hereinafter, also referred to as a "permeation rate of the product") in a case where the product permeates the separation membrane.

The membrane separation treatment by the tangential filtration method is an effective means for reducing the damage to cells and suppressing the decrease in the permeation rate of the product. By adopting the membrane separation treatment by the tangential filtration method, it is possible to reduce damage caused by cells being strongly pressed against a membrane and reduce inhibition of permeation of a product by fine particles, compared to a case where the membrane separation treatment is performed by a dead-end method. However, even in a case where the membrane separation treatment by the tangential filtration method is adopted, taking no countermeasures results in a long culturing time and a decrease in the permeation rate of the product. The detailed mechanism for the decrease in the permeation rate of the product through the separation membrane has not been clarified so far.

In the product production method according to the present disclosure, the number density Nd of fine particles (specific fine particles) which have a particle size of 8 Dp to 30 Dp in the cell suspension and are other than live cells is within a range of Expression (1). The fine particles having a particle size of 8 Dp to 30 Dp other than live cells play a significant role in the decrease in the permeation rate of the product. Particles having a particle size of smaller than 8 Dp have a smaller particle size compared to the pore diameter of the separation membrane and contribute less to the decrease in the permeation rate of the product. In a case of particles having a particle size of greater than 30 Dp, the product can pass through a gap between the particles, and thus the particles also contribute less to the decrease in the permeation rate of the product. Therefore, it is considered that the specific fine particles play a major role in the decrease in the permeation rate of the product. Such specific fine particles are generated, for example, as dead cells or fragments of damaged cells.

As a value of S/Vf is smaller, the volume of the primary side flow path of the separation membrane per unit area of the membrane surface of the separation membrane is larger. In a case where the number densities of the specific fine particles are the same, an increase in the volume of the primary side flow path of the separation membrane per unit area of the membrane surface of the separation membrane means that an increase in the number of specific fine particles, which may inhibit the permeation of the product, per unit area of the membrane surface of the separation membrane.

In addition, in a case where Dp is increased, a size of the specific fine particle is also increased, and thus a cross-sectional area per one specific fine particle is considered to be approximately proportional to $Dp^2$. It is considered that in a case where a ratio of the cross-sectional area of the specific fine particles to the area of the membrane surface of the separation membrane is increased, the permeation of the product through the separation membrane is likely to be inhibited accordingly.

For example, in a case where the cell suspension volume in the culture vessel is 1 L, S is preferably 0.005 $m^2$ to 1 $m^2$, more preferably 0.007 $m^2$ to 0.5 $m^2$, and still more preferably 0.01 $m^2$ to 0.3 $m^2$. Here, a preferred range of S is usually proportional to the cell suspension volume. That is, these preferred ranges can be interpreted as a preferred range of S per 1 L of cell suspension volume.

Furthermore, for example, in a case where the cell suspension volume is 1 L, Vf is preferably 10 $cm^3$ to 50 $cm^3$, more preferably 20 $cm^3$ to 40 $cm^3$, and still more preferably 25 $cm^3$ to 35 $cm^3$. Here, a preferred range of Vf is usually proportional to the cell suspension volume. That is, these preferred ranges can be interpreted as a preferred range of Vf per 1 L of cell suspension volume.

Therefore, in Expression (1), the number density Nd of the specific fine particles is controlled to fall within a specific range obtained based on the live cell concentration Nc, the filtration area S, the volume Vf of the primary side flow path of the separation membrane, and the pore diameter Dp of the separation membrane. In Expression (1), Nd is defined as Nc to $S/(32 \times \pi \times Vf \times Dp^2)$, but Nd is preferably $1.1 \times Nc$ to $S/(48 \times \pi \times Vf \times Dp^2)$, more preferably $1.2 \times Nc$ to $S/(64 \times \pi \times Vf \times Dp^2)$, and still more preferably $1.3 \times Nc$ to $S/(96 \times \pi \times Vf \times Dp^2)$ particles/mL.

By controlling the number density of the specific fine particles other than live cells, which inhibit the permeation of the product through the separation membrane in the culture vessel, to be within the range of Expression (1), a high permeation rate of the product can be achieved. Examples of a means for performing such control include culturing under appropriate conditions such that the number density of the specific fine particles is controlled to be within the above range.

However, in general, there is a tendency of prolongation of the culturing time and increase in the number density of the specific fine particles. Therefore, the control for maintaining the state where Expression (1) is satisfied may be performed by a treatment for removing the specific fine particles from the culture vessel.

For example, a treatment for removing the specific fine particles contained in the cell suspension may be performed by a centrifugal separation method. For example, a treatment for feeding some of the cell suspension from the culture vessel to a centrifugal separator, removing the specific fine particles by sedimentation, and returning a supernatant liquid to the culture vessel may be performed.

Alternatively, the control for maintaining the state where Expression (1) is satisfied may be performed by the bleeding step (hereinafter, also simply referred to as "bleeding") of extracting the cell suspension from the culture vessel and adding the same amount of the fresh medium as an extracted amount of the cell suspension into the culture vessel.

Since the specific fine particles which are contained in the cell suspension extracted in the bleeding step are removed from the culture vessel, the number of the specific fine particles can be reduced by the bleeding step.

In the bleeding step, the expression of "adding the same amount of the fresh medium as an extracted amount of the cell suspension into the culture vessel" means that the amount of the cell suspension in the culture vessel is maintained substantially constant as described above.

In particular, in a case where the extraction of the cell suspension is continuously performed for a separation operation by the tangential filtration method, the amount of the fresh medium supplied into the culture vessel also supplements the amount of the liquid lost as the permeated liquid, and thus the amount of the fresh medium added during the extraction in the bleeding step is not necessarily the same as the amount (hereinafter, also referred to as a bleeding amount) of the cell suspension extracted in the bleeding step.

In the bleeding step, "adding the same amount of the fresh medium as an extracted amount of the cell suspension into the culture vessel" may be performed in parallel with extracting the cell culture solution from the culture vessel, or may be performed within a predetermined time after the cell culture solution is extracted from the culture vessel.

The predetermined time may be, for example, within 10 hours, within 1 hour, within 10 minutes, or within 1 minute.

In a case where the amount of the cell suspension in the culture vessel is temporarily decreased immediately after the extraction of the cell culture solution in the bleeding step, but the amount of the cell suspension in the culture vessel is recovered to a substantially constant amount, compared to the amount of the cell suspension before the extraction, within a predetermined time, it is assumed that "adding the same amount of the fresh medium as an extracted amount of the cell suspension into the culture vessel" has been done.

The extraction in the bleeding step may be performed, from a pipe (hereinafter, also referred to as a discharge pipe) provided below a liquid level of the cell suspension in the culture vessel, simply by opening a valve provided in the middle of the discharge pipe, or may be performed by connecting the discharge pipe provided below the liquid level of the cell suspension to a pump and operating the pump.

The bleeding amount may be constant during the culture period, or may be changed in the middle of the culture period.

In a case where the bleeding amount is changed, the bleeding amount may be reset for each bleeding, or the bleeding amount may be reset every fixed period, for example, once a day. Moreover, the bleeding step may be performed intermittently or continuously.

In a case where the bleeding step is continuously performed, for example, the bleeding amount per unit time may be constant or may be changed in the middle of the culture period, the bleeding amount per unit time may be reset for each bleeding, or the bleeding amount per unit time may be reset every fixed period, for example, once a day.

Even in a case where the bleeding step is performed intermittently, it is preferable that the bleeding step is performed at least once a day. By setting the interval between the bleeding steps to be within one day, the number density of the specific fine particles can be controlled more accurately, and the decrease in the permeation rate of the product can be suppressed more effectively.

In a case where the bleeding amount or the bleeding amount per unit time is changed, in order to maintain the state where Expression (1) is satisfied for a long time, it is preferable that the number density of the specific fine particles is measured and the bleeding amount or the bleeding amount per unit time is determined based on the results, that is, that feedback control is performed, but the measurement of the number density of the specific fine particles is not essential. By appropriately setting the culture conditions, it is possible to maintain the state where Expression (1) is satisfied for at least a certain period in the culture period without performing feedback control.

That is, in the present disclosure, the control for maintaining the state where Expression (1) is satisfied does not necessarily require changing conditions such as bleeding according to the number of specific fine particles during the culture period.

However, it cannot be said that it is always possible to appropriately set a bleeding amount or a bleeding amount per unit time so as to maintain the state where Expression (1) is satisfied for a long time, only by initial setting of the bleeding amount or the bleeding amount per unit time. For example, the culture period is prolonged and the number of the specific fine particles is increased or decreased, and thus the number density may fall outside the range of Expression (1). Therefore, it is preferable that during the culture period, the feedback control is performed based on the measured value of the number density of the specific fine particles.

As described above, from the viewpoint that the state where Expression (1) is satisfied is maintained, a treatment for removing the specific fine particles from the culture vessel, such as centrifugal separation or bleeding, is preferably performed under appropriate conditions while monitoring the number density of the specific fine particles. The monitoring is not necessary to be performed at all times and may be performed, for example, immediately before each treatment for removing the specific fine particles.

Furthermore, the treatment for removing the specific fine particles may be continuously performed, and in this case, the monitoring may be performed at a predetermined timing.

Generally, in a case where the culture is performed, the cell concentration is frequently measured, but an increased amount of the specific fine particles is not proportional to the proliferation of the cells, and thus the number density of the specific fine particles cannot be known only by measuring the cell concentration. Therefore, by performing a treatment for monitoring the number density of the specific fine particles and removing the specific fine particles from the culture vessel based on the results, that is, a feedback treatment, the number density of the specific fine particles can be more reliably maintained within the range of Expression (1).

In the product production method according to the present disclosure, it is not necessary to maintain the state where Expression (1) is satisfied over the entire culture period from the initial seeding. That is, the product production method according to the present disclosure also includes an embodiment in which the state where Expression (1) is satisfied is maintained for a part of the culture period. In a case where there is a period in which Expression (1) is not satisfied, the step of culturing a cell which produces a product, the separation treatment step, the step of returning the return liquid to the culture vessel, the step of supplying a fresh medium into the culture vessel, and the step of collecting a product are performed, and the product production method according to the present disclosure is performed for the period in which Expression (1) is satisfied.

However, it is preferable that the state where Expression (1) is satisfied is maintained at least during a main production period of the product in the culture period.

Therefore, it is preferable that the state where Expression (1) is satisfied is maintained at least during a culture period after the cell concentration in the cell suspension reaches a maximum live cell concentration or a set live cell concentration. The maximum live cell concentration is the maximum value of the live cell concentration during the culture period, and varies depending on culture conditions. The set live cell concentration is a live cell concentration which is optionally set to determine a starting point for starting an operation for controlling the number of the specific fine particles by the treatment for removing the specific fine particles from the culture vessel so as to maintain the state where Expression (1) is satisfied, such as bleeding.

In a case where the cells are cultured at high density, the effect of suppressing the decrease in the permeation rate of the product by the product production method according to the present disclosure is more remarkable, and thus the set live cell concentration may be, for example, a value within a range of $2 \times 10^7$ cells/mL to $20 \times 10^7$ cells/mL. The set live cell concentration is more preferably $3 \times 10^7$ cells/mL to $15 \times 10^7$ cells/mL and still more preferably $4 \times 10^7$ cells/mL to $12 \times 10^7$ cells/mL. In a period after the live cell concentration is increased to $2 \times 10^7$ cells/mL or higher, a generation rate of the specific fine particles is also increased. Therefore, in a case where the set live cell concentration is $2 \times 10^7$ cells/mL to $20 \times$

$10^7$ cells/mL, the effect of suppressing the decrease in the permeation rate of the product is more remarkable by controlling the number of the specific fine particles to satisfy Expression (1) in the culture after reaching the set live cell concentration.

It is more preferable that during a culture period after the cell concentration in the cell suspension reaches the maximum live cell concentration or the set live cell concentration, the state where Expression (1) is satisfied is maintained at all times until the culture is completed. Moreover, it is still more preferable that the state where Expression (1) is satisfied is maintained at all times during the culture period after seeding the cells.

The control for maintaining the state where Expression (1) is satisfied is significantly different from control for keeping the concentration of the live cells or the ratio of the live cells in the cell suspension constant, for example. For example, since the specific fine particles may be cell contents released from dead cells, even in a period in which the concentration of the live cells is decreased or a period in which the ratio of the live cells is decreased, the number density of the specific fine particles may be increased. As described above, the control for maintaining the state where Expression (1) is satisfied is a technical idea different from the control for keeping the concentration of the live cells or the ratio of the live cells constant, and in the control for maintaining the state where Expression (1) is satisfied, the concentration of the live cells is usually variable.

As described above, since the number density of the specific fine particles is not proportional to the concentration of the live cells, the control for maintaining the state where Expression (1) is satisfied is also clearly different from the control for keeping an amount of a fresh medium supplied per one live cell constant in the perfusion culture. In a case where the control for keeping the amount of the fresh medium supplied per one live cell constant in the perfusion culture is performed, the state of the live cells can be stabilized, but the number density of the specific fine particles cannot be maintained within the range of Expression (1).

As described above, the step of culturing a cell which produces a product, the separation treatment step, the step of returning the return liquid to the culture vessel, the step of supplying a fresh medium into the culture vessel, and the step of collecting a product are performed, and the product production method according to the present disclosure is performed for the period in which Expression (1) is satisfied.

During a period in which each of the above steps is performed, it is preferable that the state where Expression (1) is satisfied is maintained as long as possible. By maintaining the state where Expression (1) is satisfied for a long time, it is possible to suppress the decrease in the permeation rate of the product through the separation membrane for a longer period of time.

Therefore, a duration of culture satisfying Expression (1) is preferably 3 days or longer and more preferably 10 days or longer. By continuing the culture 3 days or longer, the effect of suppressing the decrease in the permeation rate of the product is more remarkable. As the duration of the culture satisfying Expression (1) is longer, the product can be more efficiently produced for a long period of time. The upper limit of the duration of the culture satisfying Expression (1) is not particularly limited, but in a case where the culture period is too long, product production activity of the cells may be reduced. Therefore, the duration of the culture satisfying Expression (1) is more preferably 3 days to 90 days, still more preferably 10 days to 60 days, even more preferably 10 days to 40 days, and even still more preferably 15 days to 30 days.

In addition, regarding a plurality of ranges described for each parameter in the present disclosure, a new range defined by freely combining the upper limit value of one range and the lower limit value of another range as long as no contradiction occurs is also within the scope of the matters disclosed in the present disclosure. Furthermore, after seeding, there may be a period other than the period in which the product production method according to the present disclosure is performed, and for example, there may be a period for a while (for example, 1 day to 5 days) after seeding and a period in which the separation treatment step, the step of returning the return liquid to the culture vessel, and the step of supplying a fresh medium into the culture vessel are not performed.

Whether or not a state is the state where Expression (1) is satisfied can be confirmed by sampling the cell culture solution from the culture vessel and measuring the number density of the specific fine particles.

The duration of the culture satisfying Expression (1) may be determined, for example, by seeding the cells in the medium, then measuring the number density of the specific fine particles from the start of the separation treatment step, and confirming whether or not a state is the state where Expression (1) is satisfied.

In a case where the product production method according to the present disclosure further includes the bleeding step, the duration may be determined by measuring the number density of the specific fine particles from the start of the first bleeding step and confirming whether or not a state is the state where Expression (1) is satisfied. In a case where the number density of the specific fine particles falls outside the range of Expression (1), although the culturing operation is not completed, a period in which the culture satisfying Expression (1) is continued ends at the time of falling outside the range of Expression (1).

The state where Expression (1) is satisfied may be continued for 3 days or longer, for example, 10 days or longer from the start of the separation treatment step. The state where Expression (1) is satisfied may be continued, for example, for 3 days to 90 days, 10 days to 60 days, 10 days to 40 days, or 15 days to 30 days from the start of the separation treatment step. In a case where the bleeding step is started (intermittently or continuously) after the start of the separation treatment, the state where Expression (1) is satisfied may be continued for 3 days or longer, for example, 10 days or longer from the start of the first bleeding step. The state where Expression (1) is satisfied may be continued, for example, for 3 days to 90 days, 10 days to 60 days, 10 days to 40 days, or 15 days to 30 days from the start of the first bleeding step.

As described above, the effect of suppressing the decrease in the permeation rate of the product by the product production method according to the present disclosure is more remarkable in a case where the cells are cultured at high density. Therefore, the live cell concentration Nc in the cell suspension preferably satisfies Expression (2).

$$2 \times 10^7 \text{ cells/mL} \leq \text{Nc} \leq 20 \times 10^7 \text{ cells/mL} \quad \text{Expression (2)}$$

The live cell concentration Nc in the cell suspension is more preferably $3 \times 10^7$ cells/mL to $15 \times 10^7$ cells/mL and still more preferably $4 \times 10^7$ cells/mL to $12 \times 10^7$ cells/mL. In a case where the live cell concentration Nc is $2 \times 10^7$ cells/mL to $20 \times 10^7$ cells/mL, unless the number of the specific fine particles is controlled so as to satisfy Expression (1), the permeation rate of the product is likely to be decreased, but according to the product production method according to the present disclosure, in which the live cell concentration Nc satisfies Expression (1) for the number of the specific fine particles, the effect of suppressing the decrease in the permeation rate of the product can be sufficiently obtained.

It is preferable that the product production method according to the present disclosure further includes a bleeding step of extracting the cell suspension from the culture vessel and adding the same amount of the fresh medium as an extracted amount of the cell suspension into the culture vessel, in which the bleeding step includes measuring the number density Nd of the specific fine particles in the cell suspension, and adjusting a bleeding amount such that Nd satisfies Expression (1). The extraction of the cell suspension mentioned here is extraction of some of the cell suspension in the culture vessel. The number density Nd of the specific fine particles can be measured, for example, by sampling the cell suspension from the culture vessel and measuring the number density of the specific fine particles in the cell suspension. By performing so-called feedback control in which the bleeding amount is adjusted such that Nd satisfies Expression (1) based on the measured value of the number density Nd of the specific fine particles, the culture can be continued such that Expression (1) is reliably satisfied.

For example, it is preferable that in a case where the number of times Nb of bleeding of the cell suspension per day is one or more, a volume, which is expressed in a unit of L, of the cell suspension in the culture vessel is denoted by Vc, a target value, which is expressed in a unit of particles/mL, of Nd is denoted by Nd', and a number density, which is expressed in a unit of particles/mL, of specific fine particles in the cell suspension before n-th bleeding (n is any natural number which is Nb or less) is denoted by Ndn, an amount Vbn, which is expressed in a unit of L, of the n-th bleeding satisfies Expression (3) and Expression (4).

$$\text{Vbn} = \text{Vc} \times (\text{Ndn - Nd'})/\text{Ndn} \qquad \text{Expression (3)}$$

$$\text{Nc} \leq \text{Nd'} \leq \text{S}/(32 \times \pi \times \text{Vf} \times \text{Dp}^2) \quad \text{Expression (4)}$$

The target value Nd' of Nd is a number density of specific fine particles to be achieved by bleeding, and can be optionally set within a range in which Expression (4) is satisfied. By setting the amount of the n-th bleeding to the bleeding amount Vbn determined by Expression (3), the value of Nd after the n-th bleeding can be adjusted to Nd'. Nd' may be the same value every day or may be reset for each day. Moreover, a relationship between Nd' and a variable n is not described in Expression (3), but the target value Nd' of the number density of the specific fine particles may be set for each n, and in this case, Nd' can also be described as Nd'n. For example, in a case where the value of Nd after the previous bleeding is a value of Nc to $(32 \times \pi \times \text{Vf} \times \text{Dp}^2)$, the value of Nd after the previous bleeding may be used as Nd'n. Furthermore, Nb may be once or more, for example, once to 10 times, or 2 times to 5 times. As the bleeding frequency is higher, the number density of the specific fine particles tends to be controlled more precisely.

In a case where the bleeding is continuously performed, Expression (3) and Expression (4) may be applied with one bleeding from the time when the bleeding speed is changed to the time when the bleeding speed is next changed. In other words, in a case where the bleeding is continuously performed, the bleeding from the time when the bleeding speed is changed to the time when the bleeding speed is next changed constitutes one bleeding.

In a case where the bleeding is performed by opening and closing the valve provided on the discharge pipe, the bleeding amount can be adjusted by at least one of opening of the valve or an opening time of the valve, and in a case where the bleeding is performed by the pump provided on the discharge pipe, the bleeding amount can be adjusted by at least one of output of the pump or an operating time of the pump.

In Expression (4), Nd' is defined as Nc to $\text{S}/(32 \times \pi \times \text{Vf} \times \text{Dp}^2)$, but Nd' is preferably Nc $\times$ 1.1 to $\text{S}/(48 \times \pi \times \text{Vf} \times \text{Dp}^2)$, more preferably Nc $\times$ 1.2 to $\text{S}/(64 \times \pi \times \text{Vf} \times \text{Dp}^2)$, and still more preferably Nc $\times$ 1.3 to $\text{S}/(96 \times \pi \times \text{Vf} \times \text{Dp}^2)$. By setting Nd' to be equal to or higher than Nc particles/mL, it is possible to effectively obtain an effect of mitigating damage to cells caused by colliding with or getting into the membrane wall of the separation membrane, and by setting Nd' to be equal to or lower than S/($32 \times \pi \times Vf \times Dp^2$) particles/mL, it is possible to effectively obtain an effect of suppressing the decrease in the permeation rate of the product through the separation membrane.

It is preferable that in the product production method according to the present disclosure, in a case where the number of times Nb of bleeding of the cell suspension per day is one or more, a volume, which is expressed in a unit of L, of the cell suspension in the culture vessel is denoted by Vc, a target value, which is expressed in a unit of particles/mL, of Nd is denoted by Nd', and a number density, which is expressed in a unit of particles/mL, of specific fine particles in the cell suspension before n-th bleeding (n is any natural number which is Nb or less) is denoted by Ndn, an amount Vbn, which is expressed in a unit of L, of the n-th bleeding satisfies Expression (3) and Expression (5).

$$Vbn = Vc \times (Ndn - Nd')/Ndn \qquad \text{Expression (3)}$$

$$2 \times 10^7 \text{ particles/mL} \leq Nd' \leq 2 \times 10^9 \text{ particles/mL} \quad \text{Expression (5)}$$

In a case where the bleeding is continuously performed, Expression (3) and Expression (5) may be applied with one bleeding from the time when the bleeding speed is changed to the time when the bleeding speed is next changed. In other words, in a case where the bleeding is continuously performed, the bleeding from the time when the bleeding speed is changed to the time when the bleeding speed is next changed constitutes one bleeding. Vc is preferably 0.1 L to 5,000 L, more preferably 0.5 L to 3,000 L, and still more preferably 1 L to 2,000 L.

Nd' is particularly preferably set to be within a range defined by Expression (5). By setting Nd' to be within the range defined by Expression (5), it is possible to more effectively obtain the effect of mitigating damage to cells and the effect of suppressing the decrease in the permeation rate of the product. Nd' is preferably $2.2 \times 10^7$ particles/mL $\leq$ Nd' $\leq 1.5 \times 10^9$ particles/mL and more preferably $2.4 \times 10^7$ particles/mL $\leq$ Nd' $\leq 1 \times 10^9$ particles/mL.

In the product production method according to the present disclosure, the step of culturing a cell which produces a product may include stirring the cell culture solution in the culture vessel with a stirring blade. It is preferable that the step of culturing a cell which produces a product includes stirring the cell culture solution in the culture vessel with the stirring blade, in a case where a power coefficient of the stirring blade is denoted by Np, a blade diameter, which is expressed in a unit of m, of the stirring blade is denoted by Di, a rotation speed, which is expressed in a unit of $s^{-1}$, of the stirring blade is denoted by Ro, a density, which is expressed in a unit of $kg/m^3$, of the cell suspension is denoted by $\rho$, and the volume, which is expressed in a unit of L, of the cell suspension in the culture vessel is denoted by Vc, stirring is performed with a stirring power Pv, which is defined by Expression (6) and expressed in a unit of $W/m^3$, of the stirring blade in a range of 10 $W/m^3$ to 150 $W/m^3$, and in a case where a liquid level height, which is expressed in a unit of m, of the cell suspension based on a bottom of the culture vessel is denoted by Hc, a height Hb, which is expressed in a unit of m, of a bleeding extraction position is $1/2 \times$ Hc or lower. Np is preferably 0.1 to 2, more preferably 0.3 to 1.5, and still more preferably 0.5 to 1.2. Ro is preferably 1.3 $s^{-1}$ to 6.7 $s^{-1}$, more preferably 2 $s^{-1}$ to 5 $s^{-1}$, and still more preferably 3 $s^{-1}$ to 4 $s^{-1}$.

$$Pv = Np \times \rho \times Ro^3 \times Di^5/(Vc \times 10^{-3}) \qquad \text{Expression (6)}$$

The blade diameter Di is a value obtained by doubling a distance from a rotation center of the stirring blade to a point on the stirring blade farthest from the rotation center. The liquid level height Hc means a distance from the lowest point to the highest point of the cell suspension in the culture vessel, and is a value determined by a shape of the culture vessel, a liquid amount of the cell suspension, and a dead volume. Moreover, the height Hb of the bleeding extraction position is a height at which the cell suspension is extracted from the culture vessel, and is determined as a vertical distance from the lowest point of the cell suspension to a pipe intake port for bleeding. In a case where an opening surface of the pipe intake port is not horizontal, Hb is measured as a distance to a center of the opening surface of the pipe intake port.

By rotating the stirring blade, the cell suspension accommodated in the culture vessel is stirred, and thus homogeneity of the cell suspension is improved. Here, the stirring power Pv is an index indicating a degree of mixing of the cell suspension, by setting the stirring power Pv to 10 $W/m^3$ or greater, an effect of promoting substance movement between the cell and the medium tends to be effectively obtained, and setting the stirring power Pv to 150 $W/m^3$ or less, the specific fine particles can be more effectively removed by bleeding.

The stirring power Pv is preferably 10 $W/m^3$ to 150 $W/m^3$, more preferably 15 $W/m^3$ to 150 $W/m^3$, and still more preferably 20 $W/m^3$ to 100 $W/m^3$.

The height Hb of the bleeding extraction position is preferably $1/2 \times$ Hc or lower, more preferably $1/3 \times$ Hc or lower, and still more preferably $1/4 \times$ Hc or lower.

By controlling the stirring power to be within the above range and setting Hb to $1/2 \times$ Hc or lower, the specific fine particles can be more effectively removed by bleeding. This is because in a case where the stirring power is within the above range, even with stirring, the specific fine particles are appropriately settled in the culture vessel, and thus the specific fine particles can be efficiently extracted through a pipe which is for bleeding and is installed such that Hb is within the above range.

In the product production method according to the present disclosure, the step of culturing a cell which produces a product may include passing a gas through the cell suspension with a sparger.

It is preferable that the step of culturing a cell which produces a product includes passing a gas through the cell suspension with the sparger, and in a case where an interfacial tension, which is expressed in a unit of N/m, of the cell suspension is denoted by $\sigma$, a pore diameter, which is expressed in a unit of $\mu$m, of the sparger is denoted by Ds, a volume, which is expressed in a unit of mL, of a single gas generated from the sparger is denoted by Vg, a gas passage flow rate, which is expressed in a unit of mL/min, of the sparger is denoted by Q, the volume, which is expressed in a unit of L, of the cell suspension in the culture vessel is denoted by Vc, the density, which is expressed in a unit of kg/m$^3$, of the cell suspension is denoted by $\rho$, and a gravitational acceleration expressed in a unit of m/s$^2$ is denoted by g, the number Ng, which is defined by Expression (7) and Expression (8) and expressed in a unit of s$^{-1}$-L$^{-1}$, of passing gases per unit capacity and per unit time satisfies Expression (9).

$$Ng = Q/(Vg \times Vc \times 60) \quad \text{Expression (7)}$$

$$Vg = \pi \times Ds \times \sigma/(\rho \times g) \quad \text{Expression (8)}$$

$$100 \text{ s}^{-1} \cdot \text{L}^{-1} \leq Ng \leq 5{,}000 \text{ s}^{-1} \cdot \text{L}^{-1} \quad \text{Expression (9)}$$

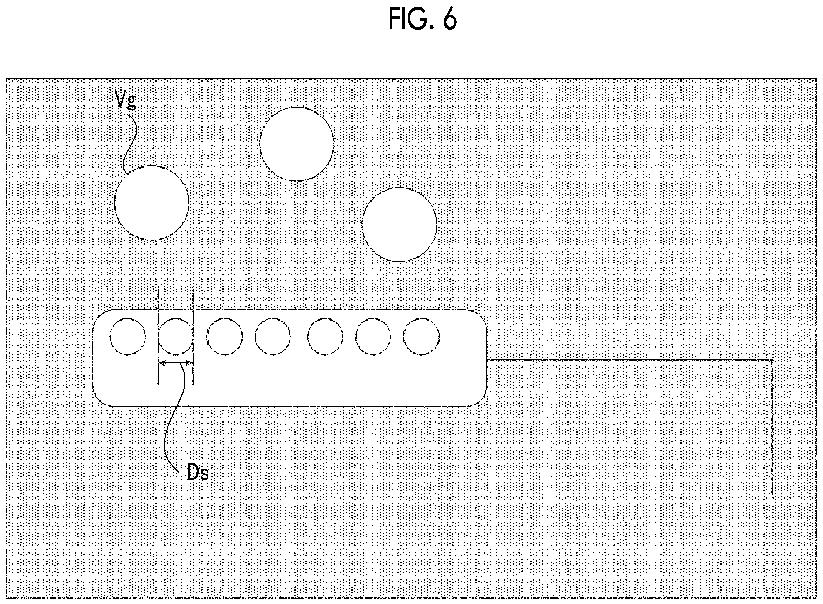

The interfacial tension $\sigma$ of the cell suspension can be measured by a plate method using an automatic surface tensiometer CBVP-Z (manufactured by Kyowa Interface Science, Inc.). The volume Vg of the single gas generated from the sparger is a volume per one bubble released from the pore of the sparger to the cell suspension as shown in Fig. 6. Vg is a theoretical value which can be determined by calculation according to Expression (8).

Therefore, each symbol in the expressions represents a characteristic in an actual cell suspension, and for example, the interfacial tension $\sigma$ represents an interfacial tension of a cell suspension at a liquid temperature and the density $\rho$ represents a density of a cell suspension at a liquid temperature. The number Ng of passing gases is a value indicating how may bubbles having the volume Vg determined by the above calculation are supplied per second per 1 L of the cell suspension. In a case where the number Ng of passing gases is 100 s$^{-1}\cdot$L$^{-1}$ or more, the effect of promoting the substance movement between the cell and the medium can be effectively obtained by passing gases from the sparger, and in a case where the number Ng of passing gases is 3,000 s$^{-1}\cdot$L$^{-1}$ or less, damage to cells due to bubble breakage of the gas is small and an effect of suppressing the number density of the specific fine particles can be efficiently obtained. As the number Ng of passing gases, which is the number of gases generated per unit volume and per unit time, is increased, the gas generated from the sparger entrains surrounding cells and breaks bubbles to promote an increase of the specific fine particles. Therefore, by controlling the number Ng of passing gases to be within the above range, the generation of the specific fine particles can be suppressed.

The number Ng of passing gases is preferably 100 s$^{-1}\cdot$L$^{-1}$ to 5,000 s$^{-1}\cdot$L$^{-1}$, more preferably 200 s$^{-1}\cdot$L$^{-1}$ to 3,500 s$^{-1}\cdot$L$^{-1}$, and most preferably 300 s$^{-1}\cdot$L$^{-1}$ to 2,000 s$^{-1}\cdot$L$^{-1}$.

The interfacial tension $\sigma$ of the cell suspension and the pore diameter Ds of the sparger are preferably in the following ranges, respectively.

$\sigma$ is preferably 10 mN/m to 100 mN/m, more preferably 20 mN/m to 80 mN/m, and still more preferably 30 mN/m to 60 mN/m.

Ds is preferably 0.1 $\mu$m to 200 $\mu$m, more preferably 0.2 $\mu$m to 100 $\mu$m, and still more preferably 0.5 $\mu$m to 50 $\mu$m.

In a case where $\sigma$ is 10 mN/m or greater and Ds is 0.1 $\mu$m or greater, the effect of promoting the substance movement between the cell and the medium can be effectively produced. In a case where $\sigma$ is $10.0 \times 10^{-2}$ N/m or less and Ds is 200 $\mu$m or less, damage to cells due to passing gases can be suppressed, and a sufficient number of passing gas bubbles can be generated.

It is preferable that in the product production method according to the present disclosure, the pore diameter Dp of the separation membrane is 0.1 $\mu$m to 1 $\mu$m, and during a culture period after the live cell concentration reaches a maximum live cell concentration or a set live cell concentration, Nd satisfies Expression (12) at all times.

$$2 \times 10^7 \leq Nd \leq 2 \times 10^9 \quad \text{Expression (12)}$$

Nd more preferably satisfies $2.2 \times 10^7 \leq Nd \leq 1.5 \times 10^9$ and still more preferably satisfies $2.4 \times 10^7 \leq Nd \leq 1 \times 10^9$.

In a case where the pore diameter Dp of the separation membrane is 0.1 $\mu$m or greater, the product can permeate efficiently, and in a case where the pore diameter Dp of the separation membrane is 1 $\mu$m or less, the permeation of cells can be efficiently prevented.

Furthermore, during the culture period after the live cell concentration reaches the maximum live cell concentration or the set live cell concentration, which is the main period in which the product is produced, Nd is particularly preferably set to be within a range defined by Expression (12). By setting Nd to be within the range defined by Expression (12), it is possible to more effectively obtain the effect of mitigating damage to cells and the effect of suppressing the decrease in the permeation rate of the product. Dp is preferably 0.1 $\mu$m to 1 $\mu$m, more preferably 0.15 $\mu$m to 0.8 $\mu$m, still more preferably 0.17 $\mu$m to 0.23 $\mu$m, and most preferably 0.2 $\mu$m.

It is preferable that in the product production method according to the present disclosure, a permeation rate of the product through the separation membrane, which is calculated as a proportion of a concentration of the product contained in the permeated liquid to a concentration of the product contained in the cell suspension, is 55% or greater. According to the product production method according to the present disclosure, the decrease in the permeation rate of the product through the separation membrane can be effectively suppressed, and thus the permeation rate of the product can be maintained at 55% or greater. In particular, it is more preferable that during the culture period after the live cell concentration reaches the maximum live cell concentration or the set live cell concentration, which is the main period in which the product is produced, the permeation rate of the product through the separation membrane is 55% or greater at all times. The permeation rate of the product through the separation membrane is more preferably 60% or greater, still more preferably 70% or greater, and even more preferably 80% or greater.

Hereinafter, the product production method according to the present disclosure will be described in more detail with reference to the drawings. Fig. 4 shows an example of the filter part comprising a flat membrane-type separation membrane, and Fig. 5 schematically shows an aspect of an example of the membrane separation treatment by the tangential filtration method in a case where the flat membrane-type separation membrane as shown in Fig. 4 is used. In this example, a targeted product produced by a cell which produces a product is an antibody.

In a case where the cell suspension flows in from the inlet port 30a of the filter part having the flat membrane-type separation membrane shown in Fig. 4 and the cell suspension flows out from the outlet port 30b, the cell suspension flows from the inlet port 30a toward the outlet port 30b in parallel to the membrane surface of the separation membrane. As shown in Fig. 5, the cell (white circle in the drawing) contained in the cell suspension, the antibodies (Y characters in the drawing), the specific fine particles (circles filled with dots in the drawing), and the like are present on the primary side of the filter part. In Fig. 5, an upper side of a separation membrane 24 is a primary side and a lower side is a secondary side.

Since the cell has a size larger than the pore diameter Dp of the separation membrane, the cell cannot permeate the separation membrane and remains on the primary side of the separation membrane. Since the antibodies have a size smaller than the pore diameter Dp of the separation membrane, the antibodies are transferred to the secondary side through the pores of the separation membrane. Some of the specific fine particles are located between the cell and the separation membrane, and thus the damage to the cell caused by colliding with or getting into the membrane wall of the separation membrane is mitigated.

On the other hand, since the specific fine particles also prevent the antibodies from approaching the pores of the separation membrane, the antibodies are prevented from permeating the separation membrane, and thus the permeation rate of the antibody is decreased. In a case where the number of specific fine particles is large with respect to the filtration area S, which is membrane surface area of the separation membrane, the decrease in the permeation rate of the antibody is particularly remarkable, while it is preferable that a certain number of specific fine particles is present for cell protection.

From these facts, it is understood that by controlling the number density of the specific fine particles so as to satisfy Expression (1), the damage to the cells can be suppressed and the decrease in the permeability of the antibody can be suppressed.

In addition, in the membrane separation treatment by the tangential filtration method, in a case where a flow direction in a space on the supply side of the filter part in the tangential filtration is reversed with the lapse of time, that is, the flow is reciprocated, the outlet port 30b and the inlet port 30a in Fig. 4 are reversed each time the flow direction is reversed.

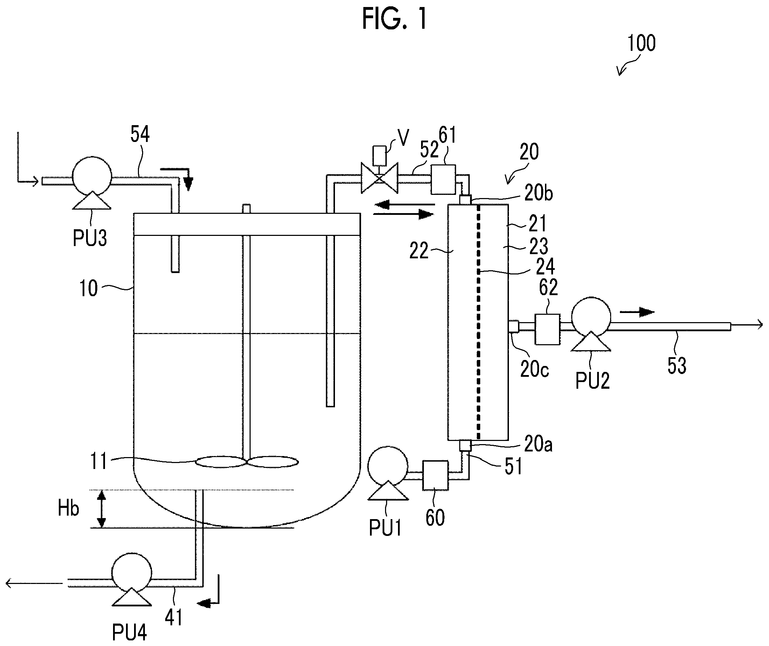

Fig. 1 is a diagram showing an example of a configuration of a cell culture apparatus 100 applicable to implementation of the product production method according to the present disclosure.

The cell culture apparatus 100 includes: a culture vessel 10 for accommodating and culturing a cell, which produces an antibody as an example of a product, together with a medium; a flow path 52 for extracting a cell suspension accommodated in the culture vessel 10 and feeding the cell suspension to a filter part 20; the filter part 20 having a separation membrane 24 for subjecting the cell suspension extracted from the culture vessel 10 to a membrane separation treatment by the tangential filtration method; a flow path 51 for discharging components blocked by the separation membrane 24 from the filter part 20; and a flow path 53 for recovering components (permeated liquid) permeating the separation membrane 24.

A stirring device having a stirring blade 11 is provided inside the culture vessel 10. By rotating the stirring blade 11, the medium accommodated in the culture vessel 10 is stirred, and thus homogeneity of the medium is maintained.

One end of the flow path 51 is connected to an outlet/inlet port 20a of the filter part 20, and the other end is connected to a pump PU1. The pump PU1 causes flow of a liquid through the flow path 51, and the cell suspension in the culture vessel 10 is extracted through the flow path 52 or flow in the opposite direction is formed. Liquid feeding pressure in the flow path 51 can be measured by a pressure gauge 60 installed between the pump PU1 and the filter part 20, liquid feeding pressure in the flow path 52 can be measured by a pressure gauge 61 which is provided on the flow path 52 and installed between a pinch valve V and the filter part 20. Liquid feeding pressure P of the pump PU1 can be adjusted by opening of the pinch valve V.

The filter part 20 comprises a vessel 21, and the separation membrane 24 which separates a space inside the vessel 21 into a supply side 22 and a permeation side 23 and performs the membrane separation treatment on the cell suspension extracted from the culture vessel 10. Moreover, the filter part 20 has, on the supply side 22, the outlet/inlet port 20a through which the cell suspension flows out or flows in, and an inlet/outlet port 20b through which the cell suspension flows in or flows out. The cell suspension extracted from the culture vessel 10 passes over the separation membrane 24 while flowing into the vessel 21 from the inlet/outlet port 20b and flowing out of the vessel 21 from the outlet/inlet port 20a. The filter part 20 performs the membrane separation treatment by the tangential filtration method in which components having a small size is fed to the permeation side 23 while flowing the liquid to be subjected to the membrane separation treatment along the membrane surface of the separation membrane 24 (in a direction parallel to the membrane surface). The membrane separation treatment by the tangential filtration method may form flow in which the cell suspension extracted from the culture vessel 10 circulates in one direction parallel along the membrane surface of the separation membrane 24, or may form flow in which the cell suspension reciprocates along the membrane surface of the separation membrane 24, but here, a case where reciprocating flow is formed will be mainly described.

The cell contained in the cell suspension does not permeate the separation membrane 24 and flows out of the vessel 21 from the outlet/inlet port 20a, and the liquid containing the cell is once fed to the pump PU1 via the flow path 51. The pump PU1 is a diaphragm pump, and this liquid is temporarily stored in a space formed by movement of the diaphragm. The pump PU1 is used as both a suction port and a jetting port, and thus is capable of forming the reciprocating flow by the movement of the diaphragm. On the other hand, the antibody contained in the cell suspension permeates the separation membrane 24, and is discharged from a discharge port 20c provided on the permeation side 23 to the outside of the vessel 21. The flow path 53 provided with a pump PU2 is connected to the permeation side 23, and the permeated liquid containing the antibody which has permeated the permeation side 23 is recovered via the flow path 53 (recovery step). Liquid feeding pressure in the flow path 53 can be measured by a pressure gauge 62 provided between the filter part 20 and the pump PU2. Since the cell does not permeate the separation membrane 24 but the antibody permeates the separation membrane 24, the permeated liquid has a cell concentration lower than that of the cell suspension and contains the antibody.

The antibody which has permeated the separation membrane 24 and is contained in the recovered permeated liquid is fed, via the flow path 53, to a purification treatment part (not shown) for purifying the antibody.

In the membrane separation treatment by the tangential filtration method, in a case where the flow in which the cell suspension extracted from the culture vessel 10 reciprocates along the membrane surface of the separation membrane 24 is formed, a liquid feeding direction of the pump PU1 is reversed with the lapse of time. In a case where the liquid feeding direction of the pump PU1 is reversed, the cell suspension is extracted from the pump PU1 via the flow path 51 and fed to the filter part 20, and the components blocked by the separation membrane 24 are returned as a return liquid from the filter part 20 to the inside of the culture vessel 10 via the flow path 52. Since the cell does not permeate the separation membrane 24, the return liquid has a cell concentration higher than that of the cell suspension. Moreover, the embodiment in which the reciprocating flow is formed using an internal space of the pump PU1 has been described here, but the embodiment according to the present disclosure is not limited thereto. For example, by providing an additional flow path (not shown) which connects the pump PU1 and the culture vessel 10, the liquid flowing out from the outlet/inlet port 20a may be returned as a return liquid to the culture vessel 10. In this case, the flow direction can be one direction, but as necessary, the liquid feeding direction of the pump PU1 can be reversed with the lapse of time.

The cell culture apparatus 100 has a flow path 54 for supplying a fresh medium to the culture vessel 10, and a pump PU3 provided in the middle of the flow path 54. By operating the pump PU3, a fresh medium is added into the culture vessel 10 through the flow path 54 so as to supplement the amount of the liquid lost as the permeated liquid as well as the amount of the liquid lost by bleeding.

In order to control a ratio of the specific fine particles in the culture vessel 10 to be within the range of Expression (1), bleeding is performed in which the cell suspension in the culture vessel is extracted from a flow path 41 having one end opened at a bottom in the culture vessel, and a fresh medium for supplementing a reduced amount of the cell suspension is supplied from the flow path 54. The bleeding may be performed intermittently or continuously. A pump PU4 is provided in the middle of the flow path 41, and the cell suspension is extracted from the culture vessel 10 via the flow path 41 which is opened at a potion where a height from the lowest point of the cell suspension is Hb. The extracted bleeding solution may be discarded, or may be subjected to a membrane treatment or the like to collect the product. Furthermore, the culture vessel 10 may comprise a sampling port (not shown) for collecting a sample for measuring the number density of the specific fine particles.

As described above, according to the present disclosure, there is provided a product production method which is capable of maintaining the permeation rate of the product through the separation membrane at a high level even in a case where the cell is cultured at a high concentration. The produced product can be used, for example, for a biopharmaceutical, regenerative medicine, and the like.

Examples

Hereinafter, the embodiment of the present disclosure will be more specifically described based on Examples, but the embodiment of the present disclosure is not limited to the following Examples.

A test for producing a product was conducted using the cell culture apparatus shown in Fig. 1.

First, 0.8 L of a medium (CD OptiCHO (trade name), manufactured by Thermo Fisher Scientific Solutions LLC) was put in a culture vessel, retained at 37°C, subjected to air passing at 38 mL/min and $CO_2$ passing at 2 mL/min from an upper surface of the culture vessel, and left for 1 day. Next, cells producing an antibody as a product were seeded such that a cell concentration in the culture vessel was $2.0 \times 10^5$ cells/mL and a liquid amount in the culture vessel was 1.0 L. Three days after seeding, continuous extraction of the cell suspension by the pump, filtration (separation treatment step) by the tangential filtration method, and supply (step of supplying a fresh medium) of a fresh medium (mixture of CD OptiCHO (trade name), manufactured by Thermo Fisher Scientific Solutions LLC and Cell Boost 7a and 7b (trade name), manufactured by GE Healthcare) were performed at a rate of 1.2 L/day. In the filtration by the tangential filtration method, the cell suspension was separated into a return liquid having a cell concentration higher than that of the cell suspension and a permeated liquid having a cell concentration lower than that of the cell suspension and containing an antibody as a product, and the return liquid was returned to the culture vessel (step of returning the return liquid to the culture solution). Moreover, by recovering the permeated liquid, the antibody as a product was collected.

In this state, the culture was continued until the live cell concentration reached a predetermined set live cell concentration. The set live cell concentration was $8 \times 10^7$ cells/mL in Examples 1 and 4 to 12 and Comparative Examples 1 to 6, $12 \times 10^7$ cells/mL in Example 2, and $14 \times 10^7$ cells/mL in Example 3. A day when the live cell concentration reached the predetermined set live cell concentration was set as a first day, and from the first day, the culture was continued while performing a bleeding operation (bleeding step) once a day according to protocols listed in Tables 2 and 3 (culturing step).

From the first day, respective liquids were sampled from the inside of the culture vessel and the secondary side of the separation membrane before and after the bleeding, and the number density of the specific fine particles in the culture vessel and the antibody concentrations in the culture vessel and on the secondary side of the separation membrane were each quantified. Regarding the number density of the specific fine particles and the permeation rate of the antibody for each day, an arithmetic mean of values before and after the bleeding was used. However, only on the last day of the culture, an arithmetic mean of values after the bleeding on the previous day and before the bleeding on the day was used. Moreover, the permeation rate (permeation rate of the product) of the antibody for each day was calculated as antibody concentration on secondary side of separation membrane/antibody concentration in culture vessel $\times$ 100.

The bleeding for each day was performed according to the following protocol.

<Bleeding protocol>

For Examples and Comparative Examples in which "Depending on number density of specific fine particles" is described in Tables 2 and 3, the number density of the specific fine particles before the bleeding on the day was denoted by Nd, the number density of the specific fine particles after the bleeding on the previous day was denoted by a target number density Nd', and the bleeding amount was set to (Nd - Nd')/Nd $\times$ 1 L. However, the upper limit value of Nd' was $9.5 \times 10^8$ particles/mL.

For Examples and Comparative Examples in which "Constant amount" is described in Tables 2 and 3, the bleeding amount for each day was kept constant at 0.20 L.

For Comparative Examples in which "Depending on cell concentration" is described in Tables 2 and 3, the cell concentration before the bleeding was denoted by Nc, the set live cell concentration was denoted by Nc', and the bleeding amount was set to (Nc - Nc')/Nc $\times$ 1 L.

In the above test, a diaphragm-type reciprocating ATF pump (ATF2 (trade name), manufactured by Repligen Corporation) was used as a cell suspension extraction filtration pump.

The number density of the specific fine particles was quantified by subtracting the number of live cells having a particle size of 8 Dp to 30 Dp from the total number of particles having a particle size of 8 Dp to 30 Dp, using Multisizer 4e (trade name) and Vi-Cell XR (trade name) of Beckman Coulter, Inc.

A liquid high performance chromatograph (Prominence (trade name), manufactured by Shimadzu Corporation) was used to quantify the antibody concentration. The permeated liquid on the secondary side of the separation membrane was loaded as it was. As a column, Applied Biosystems POROS 50A having an inner diameter of 4.6 mm $\times$ 50 mm (manufactured by Thermo Fisher Scientific Solutions LLC) was used. Moreover, 20 mM phosphate buffer + 300 mM NaCl (pH of 7) and 20 mM phosphate buffer + 300 mM NaCl (pH of 2.8) were used as an eluant, and the measurement was performed by a gradient elution method.

An interfacial tension of the culture solution at a liquid temperature was measured by a plate method using an automatic surface tensiometer (CBVP-Z (trade name), manufactured by Kyowa Interface Science, Inc.).

In addition, the cells used in the above test was a CHO cell producing an antibody, the liquid amount $V_c$ of the cell suspension in the culture vessel was 1.0 L, the liquid level height $H_c$ of the culture solution (cell suspension) in the culture vessel was 0.088 m. Furthermore, as the separation membrane, a polyethersulfone (PES) filter F2RF02PES of Repligen Corporation, which has a pore diameter ($D_p$) of 0.2 $\mu$m and is a hollow fiber-type MF membrane, was used. The filtration area of this separation membrane was 0.13 m$^2$, a hollow fiber diameter $D_f$ was $1.0 \times 10^{-3}$ m, and the volume $V_f$ of the primary side flow path was 33 cm$^3$. The blade diameter $D_i$ of the stirring blade was $85 \times 10^{-3}$ m and the power coefficient $N_p$ was 1.0. In the culture solution, that is, the cell suspension in the culture vessel, the density $\rho$ was 1,000 kg/m$^3$ and the interfacial tension $\sigma$ was $5.1 \times 10^{-2}$ N/m. The value of $S/(32 \times \pi \times V_f \times D_p^2)$ in the above test was $9.8 \times 10^8$ particles/mL.

Evaluation was performed according to the following evaluation standards by using a minimum value among the measured values of the permeation rate of the antibody during the duration of the culture.

A: The minimum permeation rate of the antibody was 85% or greater
B: The minimum permeation rate of the antibody was 75% or greater and less than 85%
C: The minimum permeation rate of the antibody was 65% or greater and less than 75%
D: The minimum permeation rate of the antibody was 55% or greater and less than 65%
E: The minimum permeation rate of the antibody was less than 55%

The results obtained by Examples and Comparative Examples are shown in Tables 2 and 3 below. Among the measured values of the number density of the specific fine particles during the duration of the culture, a minimum value and a maximum value are shown as a minimum number density and a maximum number density, respectively, in Tables 2 and 3.

Furthermore, among the measured values of the live cell concentration during the duration of the culture, a maximum value is shown as a maximum cell concentration in Tables 2 and 3.

In all of Examples and Comparative Examples, the minimum number density of the specific fine particles is a value greater than the maximum cell concentration, and thus $N_c \leq N_d$, which is inequality on a left side in Expression (1), is satisfied.

Whether or not $N_d \leq S/(32 \times \pi \times V_f \times D_p^2)$, which is inequality on a right side in Expression (1), was satisfied at all times during a 18-day period in which the culture and the filtration by the tangential filtration method were continued could be determined by comparing the maximum number density of the specific fine particles with the value of $S/(32 \times \pi \times V_f \times D_p^2)$.

Among the bleeding amounts during a duration of the culture, a minimum value and a maximum value are shown as a minimum bleeding amount and a maximum bleeding amount, respectively, in Tables 2 and 3. In a case where the bleeding protocol is "Constant amount", the bleeding amount is the same every time, and thus values of the minimum bleeding amount and the maximum bleeding amount are the same.

In Tables 2 and 3, the duration of the culture is expressed in a unit of days, the maximum cell concentration is expressed in a unit of 10$^7$ cells/mL, the minimum number density and the maximum number density of the specific fine particles are expressed in a unit of 10$^8$ particles/mL, the minimum bleeding amount and the maximum bleeding amount are expressed in a unit of L, the extraction height is expressed in a unit of m, the rotation speed of the stirring blade is expressed in a unit of s$^{-1}$, the stirring power is expressed in a unit of W/m$^3$, the pore diameter of the sparger is expressed in a unit of $\mu$m, the volume of the passing gas is expressed in a unit of 10$^{-4}$ mL, the gas passing amount is expressed in a unit of mL/min, the number of the passing gases is expressed in a unit of s$^{-1}\cdot$L$^{-1}$, and the permeation rate of the antibody is expressed in a unit of %.

[Table 2]

| Example | Culture | Cell | Specific fine particle | Specific fine particle | Bleeding | | | | Stirring blade | Stirring blade | Sparger | | | | Permeation rate of antibody | | |
|---|---|---|---|---|---|---|---|---|---|---|---|---|---|---|---|---|---|
| | Duration | Maximum cell concentration | Minimum number density | Maximum number density | Protocol | Minimum bleeding amount | Maximum bleeding amount | Extraction height | Rotation speed | Stirring power | Pore diameter | Volume of passing gas | Gas passing amount | Number of passing gases | Maximum | Minimum | Determination |
| | | $N_{c, max}$ | $N_{d, min}$ | $N_{d, max}$ | | $V_{b, min}$ | $V_{b, max}$ | $H_b$ | $R_o$ | $P_v$ | $D_s$ | $V_g$ | $Q$ | $N_g$ | | | |
| | [days] | [$\times 10^7$ cells/mL] | [$\times 10^8$ particles/mL] | [$\times 10^8$ particles/mL] | | [L] | [L] | [m] | [$s^{-1}$] | [$W/m^3$] | [$\mu m$] | [$\times 10^{-4}$ mL] | [mL/min] | [$s^{-1} \cdot L^{-1}$] | [%] | [%] | |
| Example 1 | 18 | 8.5 | 1.7 | 2.5 | Depending on number density of specific fine particles | 0.17 | 0.24 | 0.015 | 1.8 | 27 | 20 | 3.3 | 20 | 1019 | 96 | 86 | A |
| Example 2 | 18 | 12.5 | 2.9 | 3.8 | Depending on number density of specific fine particle | 0.17 | 0.26 | 0.015 | 1.8 | 27 | 20 | 3.3 | 20 | 1019 | 94 | 81 | B |
| Example 3 | 18 | 15 | 5.2 | 6.3 | Depending on number density of specific fine particle | 0.21 | 0.27 | 0.015 | 1.8 | 27 | 20 | 3.3 | 20 | 1019 | 78 | 66 | C |
| Example 4 | 18 | 8.5 | 2.1 | 4.7 | Constant amount | 0.2 | 0.2 | 0.015 | 1.8 | 27 | 20 | 3.3 | 20 | 1019 | 96 | 75 | B |
| Example 5 | 18 | 8.5 | 2.3 | 5.4 | Constant amount | 0.2 | 0.2 | 0.028 | 1.8 | 27 | 20 | 3.3 | 20 | 1019 | 96 | 71 | C |
| Example 6 | 18 | 8.5 | 2.3 | 7.8 | Constant amount | 0.2 | 0.2 | 0.042 | 1.8 | 27 | 20 | 3.3 | 20 | 1019 | 96 | 56 | D |

| Example | Culture | Cell | Specific fine particle | Specific fine particle | Bleeding | | | | Stirring blade | Stirring blade | Sparger | | | | Permeation rate of antibody | | |
|---|---|---|---|---|---|---|---|---|---|---|---|---|---|---|---|---|---|
| | Duration | Maximum cell concentration | Minimum number density | Maximum number density | Protocol | Minimum bleeding amount | Maximum bleeding amount | Extraction height | Rotation speed | Stirring power | Pore diameter | Volume of passing gas | Gas passing amount | Number of passing gases | Maximum | Minimum | Determination |
| | | Nc, max | Nd, min | Nd, max | | Vb, min | Vb, max | Hb | Ro | Pv | Ds | Vg | Q | Ng | | | |
| | [days] | [$\times 10^7$ cells/mL] | [$\times 10^8$ particles/mL] | [$\times 10^8$ particles/mL] | | [L] | [L] | [m] | [s$^{-1}$] | [W/m$^3$] | [$\mu$m] | [$\times 10^{-4}$ mL] | [mL/min] | [s$^{-1}\cdot$L$^{-1}$] | [%] | [%] | |
| Example 7 | 18 | 8.5 | 2.5 | 5.7 | Depending on number density of specific fine particle | 0.21 | 0.28 | *0.055* | 1.8 | 27 | 20 | 3.3 | 20 | 1019 | 94 | 68 | C |
| Example 8 | 18 | 8.5 | 1.9 | 5.3 | Constant amount | 0.2 | 0.2 | 0.015 | 3 | 120 | 20 | 3.3 | 20 | 1019 | 97 | 72 | C |
| Example 9 | 18 | 8.5 | 2.2 | 7.6 | Constant amount | 0.2 | 0.2 | 0.015 | 3.3 | 164 | 20 | 3.3 | 20 | 1019 | 96 | 57 | D |
| Example 10 | 18 | 8.5 | 2.3 | 6 | Depending on number density of specific fine particle | 0.22 | 0.25 | 0.015 | 3.8 | 234 | 20 | 3.3 | 20 | 1019 | 95 | 67 | C |
| Example 11 | 18 | 8.5 | 3.4 | 4.5 | Depending on number density of specific fine particle | 0.18 | 0.24 | 0.015 | 1.8 | 27 | 20 | 3.3 | 40 | 2039 | 87 | 77 | B |
| Example 12 | 18 | 8.5 | 6.6 | 8.3 | Depending on number density of specific fine particle | 0.19 | 0.26 | 0.015 | 1.8 | 27 | 20 | 3.3 | 70 | 3568 | 67 | 57 | D |

[Table 3]

| Comparative example | Culture | Cell | Specific fine particle | Specific fine particle | Bleeding | | | | | | Stirring blade | Stirring blade | Sparger | | | | Permeation rate of antibody | | |
|---|---|---|---|---|---|---|---|---|---|---|---|---|---|---|---|---|---|---|---|
| | Duration Duration | Maximum cell concentration | Minimum number density | Maximum number density | Protocol | Minimum bleeding amount | Maximum bleeding amount | Extraction height | Rotation speed | Stirring power | Pore diameter | Volume of passing gas | Gas passing amount | Number of passing gases | Maximum | Minimum | Determination |
| | | $N_{c, max}$ | $N_{d, min}$ | $N_{d, max}$ | | $V_{b, min}$ | $V_{b, max}$ | $H_b$ | $R_o$ | $P_v$ | $D_s$ | $V_g$ | $Q$ | $N_g$ | | | |
| | [days] | [$\times 10^7$ cells/mL] | [$\times 10^8$ particles/mL] | [$\times 10^8$ particles/mL] | | [L] | [L] | [m] | [$s^{-1}$] | [W/m$^3$] | [$\mu$m] | [$\times 10^{-4}$ mL] | [mL/min] | [$s^{-1} \cdot L^{-1}$] | [%] | [%] | |
| Comparative example 1 | 18 | 8.5 | 2.5 | 11 | Constant amount | 0.2 | 0.2 | 0.055 | 1.8 | 27 | 20 | 3.3 | 20 | 1019 | 95 | 38 | E |
| Comparative example 2 | 18 | 8.5 | 2.3 | 10 | Constant amount | 0.2 | 0.2 | 0.015 | 3.8 | 234 | 20 | 3.3 | 20 | 1019 | 95 | 43 | E |
| Comparative example 3 | 18 | *8.5* | 7.4 | 10 | Depending on number density of specific fine particles | 0.24 | 0.3 | 0.015 | 1.8 | 27 | 2 | 0.33 | 10 | 5097 | 61 | 51 | E |
| Comparative example 4 | 18 | 8.5 | 2 | 12 | Depending on cell concentration | 0.15 | 0.23 | 0.042 | 1.8 | 27 | 20 | 3.3 | 20 | 1019 | 96 | 30 | E |
| Comparative example 5 | 18 | 8.5 | 2.3 | 11 | Depending on cell concentration | 0.16 | 0.24 | 0.015 | 3.3 | 164 | 20 | 3.3 | 20 | 1019 | 95 | 34 | E |
| Comparative example 6 | 18 | 8.5 | 7.2 | 14 | Depending on cell concentration | 0.15 | 0.24 | 0.015 | 1.8 | 27 | 20 | 3.3 | 70 | 3568 | 67 | 20 | E |

22

As shown in Tables 2 and 3, in Examples 1 to 12 in which Expression (1) was satisfied, the permeation rate of the antibody was as good as 65% or greater throughout the culture period of 18 days. On the other hand, in Comparative Examples 1 to 6 in which the number density fell outside of the range of Expression (1) in the middle of the culture period, the minimum value of the permeation rate of the antibody was as low as less than 55%.

From these results, it is found that the permeation rate of the product can be maintained at a high level by maintaining the state where Expression (1) is satisfied.

In addition, from the comparison between the result of Example 1 and the result of Example 4, it is found that in a case where the bleeding step includes measuring the number density Nd of the specific fine particles and adjusting the bleeding amount such that Nd satisfies Expression (1), the decrease in the permeation rate of the product is more effectively suppressed.

On the other hand, it is found that in a case where the bleeding was performed to keep the cell concentration constant, the decrease in the permeation rate of the product cannot be suppressed as shown in Comparative Examples 4 to 6.

From the comparison between Examples 4 to 6 and the comparison between Examples 1 and 7, it is found that in a case where the ratio of Hb/Hc is within the preferred range described above, the decrease in the permeation rate of the product is more effectively suppressed.

From the comparison between Examples 8 and 9 and the comparison between Examples 1 and 10, it is found that in a case where the stirring power Pv of the rotor blade is within a range of 10 $W/m^3$ to 150 $W/m^3$, the decrease in the permeation rate of the product is more effectively suppressed.

From the comparison between Examples 1, 11, and 12, it is found that in a case where the number Ng of passing gases is within the preferred range described above, the decrease in the permeation rate of the product is more effectively suppressed.

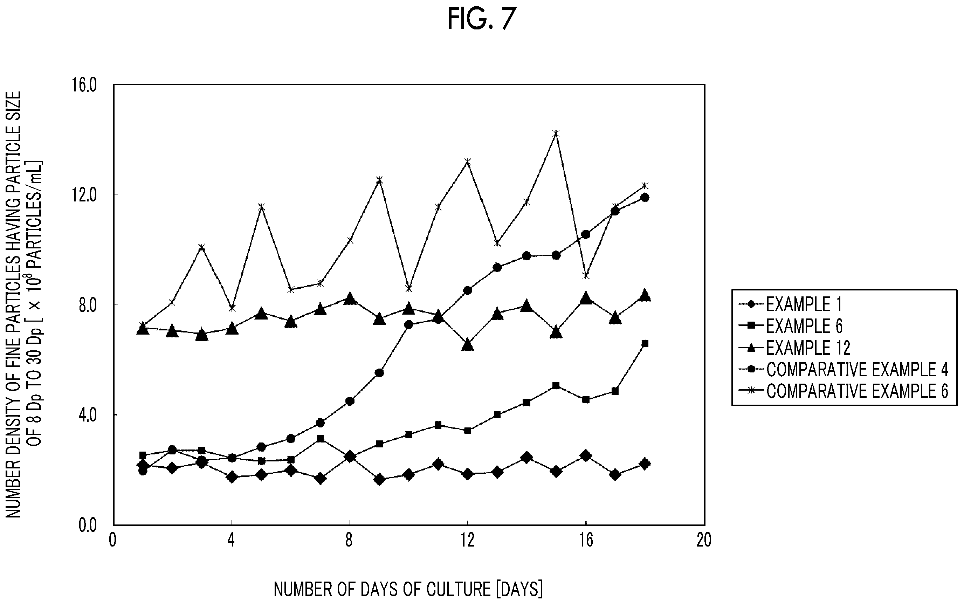

Fig. 7 shows a relationship between the number of days of culture and the number density Nd of the fine particles having a particle size of 8 Dp to 30 Dp other than the live cells, in Examples 1, 6, and 12 and Comparative Examples 4 and 6.

As can be seen from Fig. 7, in Examples 1, 6, and 12, the number density of the specific fine particles was maintained at a low level, but in Comparative Examples 4 and 6, the number density of the specific fine particles during the culture period was high.

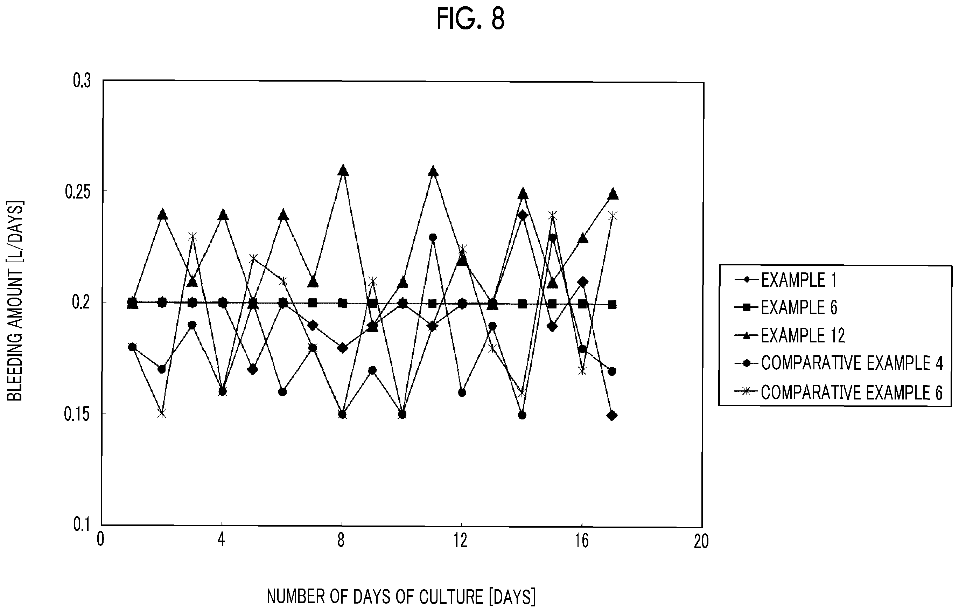

Fig. 8 shows a relationship between the number of days of culture and the bleeding amount, in Examples 1, 6, and 12 and Comparative Examples 4 and 6.

In Comparative Examples 4 and 6, the bleeding amount was adjusted according to the cell concentration, but the bleeding amount for each day was significantly different from the bleeding amount for each day in Examples 1, 6, and 12.

As described above, the adjustment of the bleeding amount according to the cell concentration is significantly different from the control of the bleeding amount for maintaining Expression (1) according to the present disclosure.

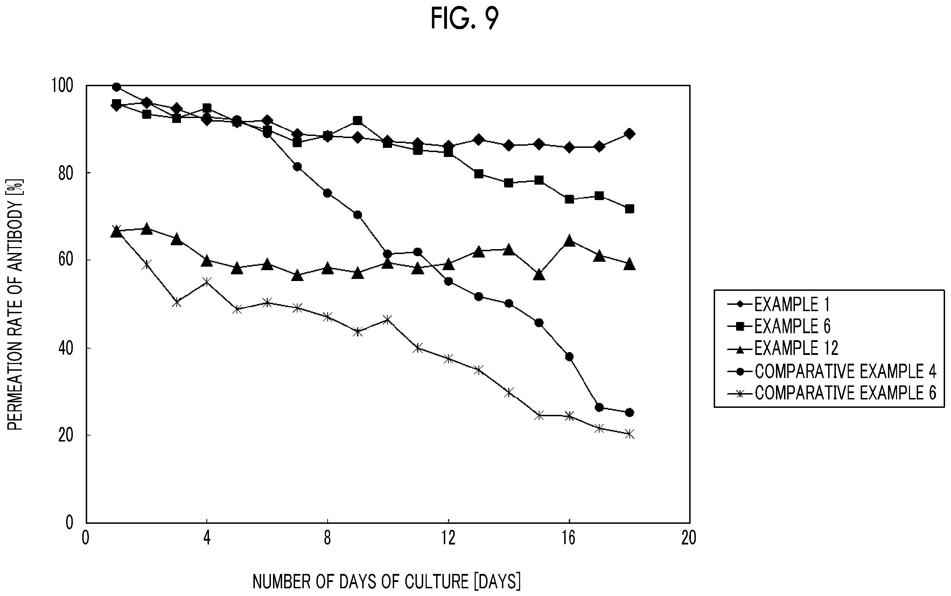

Fig. 9 shows a relationship between the number of days of culture and the permeation rate of the antibody through the separation membrane, in Examples 1, 6, and 12 and Comparative Examples 4 and 6.

As can be seen from Fig. 9, in Examples 1, 6, and 12, the decrease in the permeation rate of the antibody was effectively suppressed, but in Comparative Examples 4 and 6, the permeation rate of the antibody was significantly decreased during the culture period.

(Explanation of References)

10: Culture vessel, 11: Stirring blade, 20: Filter part, 20a: Outlet/inlet port, 20b: Outlet/inlet port, 20c: Discharge port, 21: Vessel, 22: Supply side, 23: Permeation side, 24: Separation membrane, 30a: Inlet port, 30b: Outlet port, 41: Flow path, 51 to 54: Flow path, 60 to 62: Pressure gauge, 100: Cell culture apparatus, PU1 to PU4: Pump, V: Pinch valve, Hb: Height of extraction position, Df: Hollow fiber diameter, L: Length of hollow fiber, S: Filtration area, Vf: Volume of primary side flow path of separation membrane, Dp: Pore diameter of separation membrane, Ds: Pore diameter of sparger, and Vg: Volume of single gas.

**Claims**

1. A product production method, comprising:

   a step of culturing a cell which produces a product and is contained in a cell suspension accommodated in a culture vessel;
   a separation treatment step of extracting the cell suspension from the culture vessel, and separating the cell suspension, using a separation membrane, into a return liquid having a cell concentration higher than that of the cell suspension and a permeated liquid having a cell concentration lower than that of the cell suspension and containing the product by a tangential filtration method;
a step of returning the return liquid to the culture vessel;
a step of supplying a fresh medium into the culture vessel; and
a step of collecting the product,
wherein in a case where a live cell concentration, which is expressed in a unit of cells/mL, in the cell suspension is denoted by Nc, a pore diameter, which is expressed in a unit of m, of the separation membrane is denoted by Dp, a filtration area, which is expressed in a unit of $m^2$, of the separation membrane is denoted by S, and a volume, which is expressed in a unit of $cm^3$, of a primary side flow path of the separation membrane is denoted by Vf, a number density Nd, which is expressed in a unit of particles/mL, of fine particles having a particle size of 8 Dp to 30 Dp other than live cells in the cell suspension satisfies Expression (1),

$$Nc \leq Nd \leq S/(32 \times \pi \times Vf \times Dp^2) \quad \text{Expression (1)}.$$

2.  The production method according to claim 1,
    wherein a duration of culture satisfying Expression (1) is 10 days or longer.

3.  The production method according to claim 1 or 2,
    wherein a duration of culture satisfying Expression (1) is 10 days to 40 days.

4.  The production method according to any one of claims 1 to 3,
    wherein the live cell concentration Nc satisfies Expression (2),

$$2 \times 10^7 \text{ cells/mL} \leq Nc \leq 20 \times 10^7 \text{ cells/mL} \quad \text{Expression (2)}.$$

5.  The production method according to any one of claims 1 to 4, further comprising:

    a bleeding step of extracting the cell suspension from the culture vessel and adding the same amount of the fresh medium as an extracted amount of the cell suspension into the culture vessel,
    wherein the bleeding step includes measuring the number density Nd of the fine particles having a particle size of 8 Dp to 30 Dp other than the live cells in the cell suspension, and adjusting a bleeding amount such that Nd satisfies Expression (1).

6.  The production method according to claim 5,
    wherein in a case where the number of times Nb of bleeding of the cell suspension per day is one or more, a volume, which is expressed in a unit of L, of the cell suspension in the culture vessel is denoted by Vc, a target value, which is expressed in a unit of particles/mL, of Nd is denoted by Nd', and a number density, which is expressed in a unit of particles/mL, of fine particles having a particle size of 8 Dp to 30 Dp other than live cells in the cell suspension before n-th bleeding (n is any natural number which is Nb or less) is denoted by Ndn, an amount Vbn, which is expressed in a unit of L, of the n-th bleeding satisfies Expression (3) and Expression (4),

$$Vbn = Vc \times (Ndn - Nd')/Ndn \quad \text{Expression (3)}$$

$$Nc \leq Nd' \leq S/(32 \times \pi \times Vf \times Dp^2) \quad \text{Expression (4)}.$$

7.  The production method according to claim 5 or 6,
    wherein in a case where the number of times Nb of bleeding of the cell suspension per day is one or more, a volume, which is expressed in a unit of L, of the cell suspension in the culture vessel is denoted by Vc, a target value, which is expressed in a unit of particles/mL, of Nd is denoted by Nd', and a number density, which is expressed in a unit of particles/mL, of the fine particles having a particle size of 8 Dp to 30 Dp other than live cells in the cell suspension before n-th bleeding (n is any natural number which is Nb or less) is denoted by Ndn, the amount Vbn, which is expressed in a unit of L, of the n-th bleeding satisfies Expression (3) and Expression (5),

$$Vbn = Vc \times (Ndn - Nd')/Ndn \quad \text{Expression (3)}$$

$$2 \times 10^7 \text{ particles/mL} \leq \text{Nd'} \leq 2 \times 10^9 \text{ particles/mL} \quad \text{Expression (5)}.$$

**8.** The production method according to any one of claims 1 to 7,

wherein the step of culturing a cell which produces a product includes stirring the cell suspension in the culture vessel with a stirring blade, and
in a case where a power coefficient of the stirring blade is denoted by Np, a blade diameter, which is expressed in a unit of m, of the stirring blade is denoted by Di, a rotation speed, which is expressed in a unit of $s^{-1}$, of the stirring blade is denoted by Ro, a density, which is expressed in a unit of $kg/m^3$, of the cell suspension is denoted by $\rho$, and a volume, which is expressed in a unit of L, of the cell suspension in the culture vessel is denoted by Vc, stirring is performed with a stirring power Pv, which is defined by Expression (6) and expressed in a unit of $W/m^3$, of the stirring blade in a range of $10 \, W/m^3$ to $150 \, W/m^3$, and in a case where a liquid level height, which is expressed in a unit of m, of the cell suspension based on a bottom of the culture vessel is denoted by Hc, a height Hb, which is expressed in a unit of m, of a bleeding extraction position is $1/2 \times$ Hc or lower,

$$\text{Pv} = \text{Np} \times \rho \times \text{Ro}^3 \times \text{Di}^5/(\text{Vc} \times 10^{-3}) \quad \text{Expression (6)}.$$

**9.** The production method according to any one of claims 1 to 8,

wherein the step of culturing a cell which produces a product includes passing a gas through the cell suspension with a sparger, and
in a case where an interfacial tension, which is expressed in a unit of N/m, of the cell suspension is denoted by $\sigma$, a pore diameter, which is expressed in a unit of $\mu$m, of the sparger is denoted by Ds, a volume, which is expressed in a unit of mL, of a single gas generated from the sparger is denoted by Vg, a gas passage flow rate, which is expressed in a unit of mL/min, of the sparger is denoted by Q, a volume, which is expressed in a unit of L, of the cell suspension in the culture vessel is denoted by Vc, a density, which is expressed in a unit of $kg/m^3$, of the cell suspension is denoted by $\rho$, and a gravitational acceleration expressed in a unit of $m/s^2$ is denoted by g, the number Ng, which is defined by Expression (7) and Expression (8) and expressed in a unit of $s^{-1} \cdot L^{-1}$, of passing gases per unit capacity and per unit time satisfies Expression (9),

$$\text{Ng} = \text{Q}/(\text{Vg} \times \text{Vc} \times 60) \quad \text{Expression (7)}$$

$$\text{Vg} = \pi \times \text{Ds} \times \sigma/(\rho \times g) \quad \text{Expression (8)}$$

$$100 \, s^{-1} \cdot L^{-1} \leq \text{Ng} \leq 5{,}000 \, s^{-1} \cdot L^{-1} \quad \text{Expression (9)}.$$

**10.** The production method according to claim 9,
wherein the interfacial tension $\sigma$ of the cell suspension and the pore diameter Ds of the sparger respectively satisfy Expression (10) and Expression (11),

$$10 \text{ mN/m} \leq \sigma \leq 100 \text{ mN/m} \quad \text{Expression (10)}$$

$$0.1 \, \mu\text{m} \leq \text{Ds} \leq 200 \, \mu\text{m} \quad \text{Expression (11)}.$$

**11.** The production method according to any one of claims 1 to 10,
wherein the cell is a CHO cell.

**12.** The production method according to any one of claims 1 to 11,

wherein the pore diameter Dp of the separation membrane is $0.1 \, \mu$m to $1 \, \mu$m, and
during a culture period after the live cell concentration reaches a maximum live cell concentration or a set live cell concentration, Nd satisfies Expression (12) at all times,

$$2 \times 10^7 \le \text{Nd} \le 2 \times 10^9 \qquad \text{Expression (12)}.$$

13. The production method according to any one of claims 1 to 12,
wherein a permeation rate of the product through the separation membrane, which is calculated as a proportion of a concentration of the product contained in the permeated liquid to a concentration of the product contained in the cell suspension, is 55% or greater.

**Patentansprüche**

1. Produktproduktionsverfahren, umfassend:

   einen Schritt des Kultivierens einer Zelle, die ein Produkt produziert und in einer Zellsuspension enthalten ist, die in einem Kulturgefäß aufgenommen ist;
   einen Abtrennbehandlungsschritt des Extrahierens der Zellsuspension aus dem Kulturgefäß und des Trennens der Zellsuspension unter Verwendung einer Trennmembran in eine Rückführflüssigkeit, die eine höhere Zellkonzentration als die der Zellsuspension aufweist, und eine permeierte Flüssigkeit, die eine niedrigere Zellkonzentration als die der Zellsuspension aufweist und das Produkt enthält, durch ein Tangentialfiltrationsverfahren;
   einen Schritt des Rückführens der Rückführflüssigkeit in das Kulturgefäß;
   einen Schritt des Zuführens eines frischen Mediums in das Kulturgefäß; und
   einen Schritt des Sammelns des Produkts,
   wobei in einem Fall, in dem eine Konzentration lebender Zellen, die in einer Einheit von Zellen/mL ausgedrückt wird, in der Zellsuspension als Nc bezeichnet wird, ein Porendurchmesser, der in einer Einheit von m ausgedrückt wird, der Trennmembran als Dp bezeichnet wird, eine Filtrationsfläche, die in einer Einheit von m² ausgedrückt wird, der Trennmembran als S bezeichnet wird und ein Volumen, das in einer Einheit von cm³ ausgedrückt wird, eines Primärseiten-Strömungswegs der Trennmembran als Vf bezeichnet wird, eine Anzahldichte Nd, die in einer Einheit von Partikel/mL ausgedrückt wird, von feinen Partikeln mit einer Partikelgröße von 8 Dp bis 30 Dp, die andere als lebende Zellen in der Zellsuspension sind, Ausdruck (1) erfüllt,

$$\text{Nc} \le \text{Nd} \le S/(32 \times \pi \times \text{Vf} \times \text{Dp}^2) \qquad \text{Ausdruck (1)}.$$

2. Produktionsverfahren nach Anspruch 1,
wobei eine Dauer von Kultur, die Ausdruck (1) erfüllt, 10 Tage oder länger beträgt.

3. Produktionsverfahren nach Anspruch 1 oder 2,
wobei eine Dauer von Kultur, die Ausdruck (1) erfüllt, 10 Tage bis 40 Tage beträgt.

4. Produktionsverfahren nach einem der Ansprüche 1 bis 3,
wobei die Konzentration Nc lebender Zellen Ausdruck (2) erfüllt,

$$2 \times 10^7 \text{ Zellen/mL} \le \text{Nc} \le 20 \times 10^7 \text{ Zellen/mL} \qquad \text{Ausdruck (2)}.$$

5. Produktionsverfahren nach einem der Ansprüche 1 bis 4, ferner umfassend:

   einen Ablassschritt des Extrahierens der Zellsuspension aus dem Kulturgefäß und des Hinzufügens der gleichen Menge des frischen Mediums als eine extrahierte Menge der Zellsuspension in das Kulturgefäß,
   wobei der Ablassschritt Messen der Anzahldichte Nd der feinen Partikel mit einer Partikelgröße von 8 Dp bis 30 Dp, die andere als die lebenden Zellen in der Zellsuspension sind, und Anpassen einer Ablassmenge, so dass Nd Ausdruck (1) erfüllt, enthält.

6. Produktionsverfahren nach Anspruch 5,
wobei in einem Fall, in dem die Anzahl an Malen Nb von Ablassen der Zellsuspension pro Tag eins oder mehr ist, ein Volumen, das in einer Einheit von L ausgedrückt wird, der Zellsuspension in dem Kulturgefäß als Vc bezeichnet wird, ein Zielwert, der in einer Einheit von Partikel/mL ausgedrückt wird, von Nd als Nd' bezeichnet wird und eine Anzahldichte, die in einer Einheit von Partikel/mL ausgedrückt wird, von feinen Partikeln mit einer Partikelgröße von 8 Dp bis 30 Dp, die andere als lebende Zellen in der Zellsuspension sind, vor n-tem Ablassen (n ist eine beliebige natürliche Zahl, die Nb oder weniger ist) als Ndn bezeichnet wird, eine Menge Vbn, die in einer Einheit von L ausgedrückt wird, des n-ten Ablassens Ausdruck (3) und Ausdruck (4) erfüllt,

$$Vbn = Vc \times (Ndn - Nd')/Ndn \qquad \text{Ausdruck (3)}$$

$$Nc \leq Nd' \leq S/(32 \times \pi \times Vf \times Dp^2) \qquad \text{Ausdruck (4)}.$$

**7.** Produktionsverfahren nach Anspruch 5 oder 6,
wobei in einem Fall, in dem die Anzahl an Malen Nb von Ablassen der Zellsuspension pro Tag eins oder mehr ist, ein Volumen, das in einer Einheit von L ausgedrückt wird, der Zellsuspension in dem Kulturgefäß als Vc bezeichnet wird, ein Zielwert, der in einer Einheit von Partikel/mL ausgedrückt wird, von Nd als Nd' bezeichnet wird und eine Anzahldichte, die in einer Einheit von Partikel/mL ausgedrückt wird, von feinen Partikeln mit einer Partikelgröße von 8 Dp bis 30 Dp, die andere als lebende Zellen in der Zellsuspension sind, vor n-tem Ablassen (n ist eine beliebige natürliche Zahl, die Nb oder weniger ist) als Ndn bezeichnet wird, die Menge Vbn, die in einer Einheit von L ausgedrückt wird, des n-ten Ablassens Ausdruck (3) und Ausdruck (5) erfüllt,

$$Vbn = Vc \times (Ndn - Nd')/Ndn \qquad \text{Ausdruck (3)}$$

$$2 \times 10^7 \text{ Partikel/mL} \leq Nd' \leq 2 \times 10^9 \text{ Partikel/mL} \qquad \text{Ausdruck (5)}.$$

**8.** Produktionsverfahren nach einem der Ansprüche 1 bis 7,

wobei der Schritt des Kultivierens einer Zelle, die ein Produkt produziert, Rühren der Zellsuspension in dem Kulturgefäß mit einem Rührblatt enthält, und
in einem Fall, in dem ein Leistungskoeffizient des Rührblatts als Np bezeichnet wird, ein Blattdurchmesser, der in einer Einheit von m ausgedrückt wird, des Rührblatts als Di bezeichnet wird, eine Drehzahl, die in einer Einheit von s$^{-1}$ ausgedrückt wird, des Rührblatts als Ro bezeichnet wird, eine Dichte, die in einer Einheit von kg/m$^3$ ausgedrückt wird, der Zellsuspension als $\rho$ bezeichnet wird und ein Volumen, das in einer Einheit von L ausgedrückt wird, der Zellsuspension in dem Kulturgefäß als Vc bezeichnet wird, Rühren mit einer Rührleistung Pv, die durch Ausdruck (6) definiert ist und in einer Einheit von W/m$^3$ ausgedrückt wird, des Rührblatts in einem Bereich von 10 W/m$^3$ bis 150 W/m$^3$ durchgeführt wird und in einem Fall, in dem eine Flüssigkeitshöhe, die in einer Einheit von m ausgedrückt wird, der Zellsuspension auf der Grundlage eines Bodens des Kulturgefäßes als Hc bezeichnet wird, eine Höhe Hb, die in einer Einheit von m ausgedrückt wird, einer Ablassextraktionsposition 1/2 $\times$ Hc oder niedriger ist,

$$Pv = Np \times \rho \times Ro^3 \times Di^5/(Vc \times 10^{-3}) \qquad \text{Ausdruck (6)}.$$

**9.** Produktionsverfahren nach einem der Ansprüche 1 bis 8,

wobei der Schritt des Kultivierens einer Zelle, die ein Produkt produziert, Passieren eines Gases durch die Zellsuspension mit einem Sparger enthält, und
in einem Fall, in dem eine Grenzflächenspannung, die in einer Einheit von N/m ausgedrückt wird, der Zellsuspension als $\sigma$ bezeichnet wird, ein Porendurchmesser, der in einer Einheit von $\mu$m ausgedrückt wird, des Spargers als Ds bezeichnet wird, ein Volumen, das in einer Einheit von mL ausgedrückt wird, eines einzelnen Gases, das aus dem Sparger erzeugt wird, als Vg bezeichnet wird, eine Gasdurchflussrate, die in einer Einheit von mL/min ausgedrückt wird, des Spargers als Q bezeichnet wird, ein Volumen, das in einer Einheit von L ausgedrückt wird, der Zellsuspension in dem Kulturgefäß als Vc bezeichnet wird, eine Dichte, die in einer Einheit von kg/m$^3$ ausgedrückt wird, der Zellsuspension als $\rho$ bezeichnet wird und eine Erdbeschleunigung, die in einer Einheit von m/s$^2$ ausgedrückt wird, als g bezeichnet wird, die Anzahl Ng, die durch Ausdruck (7) und Ausdruck (8) definiert ist und in einer Einheit von s$^{-1}\cdot$L$^{-1}$ ausgedrückt wird, von durchströmenden Gasen pro Einheitskapazität und pro Zeiteinheit Ausdruck (9) erfüllt,

$$Ng = Q/(Vg \times Vc \times 60) \qquad \text{Ausdruck (7)}$$

$$Vg = \pi \times Ds \times \sigma/(\rho \times g) \qquad \text{Ausdruck (8)}$$

$$100 \ \text{s}^{-1}\cdot\text{L}^{-1} \leq Ng \leq 5.000 \ \text{s}^{-1}\cdot\text{L}^{-1} \qquad \text{Ausdruck (9)}.$$

10. Produktionsverfahren nach Anspruch 9,

wobei die Grenzflächenspannung $\sigma$ der Zellsuspension und der Porendurchmesser Ds des Spargers entsprechend Ausdruck (10) und Ausdruck (11) erfüllen,

$$10 \ \text{mN/m} \leq \sigma \leq 100 \ \text{mN/m} \qquad \text{Ausdruck (10)}$$

$$0,1 \ \mu\text{m} \leq Ds \leq 200 \ \mu\text{m} \qquad \text{Ausdruck (11)}.$$

11. Produktionsverfahren nach einem der Ansprüche 1 bis 10,
wobei die Zelle eine CHO-Zelle ist.

12. Produktionsverfahren nach einem der Ansprüche 1 bis 11,

wobei der Porendurchmesser Dp der Trennmembran 0,1 $\mu$m bis 1 $\mu$m beträgt, und
während einer Kulturperiode, nachdem die Konzentration lebender Zellen eine maximale Konzentration lebender Zellen oder eine eingestellte Konzentration lebender Zellen erreicht hat, Nd zu allen Zeiten Ausdruck (12) erfüllt,

$$2 \times 10^{7} \leq Nd \leq 2 \times 10^{9} \qquad \text{Ausdruck (12)}.$$

13. Produktionsverfahren nach einem der Ansprüche 1 bis 12,
wobei eine Permeationsrate des Produkts durch die Trennmembran, die als ein Anteil einer Konzentration des Produkts, das in der permeierten Flüssigkeit enthalten ist, zu einer Konzentration des Produkts, das in der Zellsuspension enthalten ist, berechnet wird, 55 % oder größer ist.

**Revendications**

1. Procédé de production de produit, comprenant :

une étape de culture d'une cellule qui produit un produit et est contenue dans une suspension cellulaire logée dans un récipient de culture ;
une étape de traitement de séparation consistant à extraire la suspension cellulaire du récipient de culture, et à séparer la suspension cellulaire, en utilisant une membrane de séparation, en un liquide de retour ayant une concentration cellulaire supérieure à celle de la suspension cellulaire et en un liquide perméé ayant une concentration cellulaire inférieure à celle de la suspension cellulaire et contenant le produit par un procédé de filtration tangentielle ;
une étape de retour du liquide de retour dans le récipient de culture ;
une étape d'alimentation d'un milieu frais dans le récipient de culture ; et
une étape de collecte du produit,
dans lequel dans un cas où une concentration en cellules vivantes, qui est exprimée dans une unité de cellules/mL, dans la suspension cellulaire est désignée par Nc, un diamètre de pore, qui est exprimé dans une unité de m, de la membrane de séparation est désigné par Dp, une surface de filtration, qui est exprimée dans une unité de m$^2$, de la membrane de séparation est désignée par S, et un volume, qui est exprimé dans une unité de cm$^3$, d'une voie d'écoulement côté primaire de la membrane de séparation est désigné par Vf, une densité de nombre Nd, qui est exprimée dans une unité de particules/mL, de particules fines ayant une taille de particules de 8 Dp à 30 Dp autres que des cellules vivantes dans la suspension cellulaire satisfait Expression (1),

$$Nc \leq Nd \leq S/(32 \times \pi \times Vf \times Dp^{2}) \ \text{Expression (1)}.$$

**2.** Procédé de production selon la revendication 1,
dans lequel une durée de culture satisfaisant Expression (1) est de 10 jours ou plus.

**3.** Procédé de production selon la revendication 1 ou la revendication 2,
dans lequel une durée de culture satisfaisant Expression (1) est de 10 jours à 40 jours.

**4.** Procédé de production selon l'une quelconque des revendications 1 à 3,
dans lequel la concentration en cellules vivantes Nc satisfait Expression (2),

$$2 \times 10^7 \text{ cellules/mL} \leq Nc \leq 20 \times 10^7 \text{ cellules/mL Expression (2)}.$$

**5.** Procédé de production selon l'une quelconque des revendications 1 à 4, comprenant en outre :

une étape de saignée consistant à extraire la suspension cellulaire du récipient de culture et à ajouter la même quantité du milieu frais qu'une quantité extraite de la suspension cellulaire dans le récipient de culture,
dans lequel l'étape de saignée inclut la mesure de la densité de nombre Nd des particules fines ayant une taille de particules de 8 Dp à 30 Dp autres que les cellules vivantes dans la suspension cellulaire, et l'ajustement d'une quantité de saignée de sorte que Nd satisfasse Expression (1).

**6.** Procédé de production selon la revendication 5,
dans lequel dans un cas où le nombre de fois Nb de saignée de la suspension cellulaire par jour est de un ou plus, un volume, qui est exprimé dans une unité de L, de la suspension cellulaire dans le récipient de culture est désigné par Vc, une valeur cible, qui est exprimée dans une unité de particules/mL, de Nd est désignée par Nd', et une densité de nombre, qui est exprimée dans une unité de particules/mL, de particules fines ayant une taille de particules de 8 Dp à 30 Dp autres que des cellules vivantes dans la suspension cellulaire avant n-ième saignée (n est un nombre naturel quelconque qui est Nb ou moins) est désignée par Ndn, une quantité Vbn, qui est exprimée dans une unité de L, de la n-ième saignée satisfait Expression (3) et Expression (4),

$$Vbn = Vc \times (Ndn - Nd')/Ndn \text{ Expression (3)}$$

$$Nc \leq Nd' \leq S/(32 \times \pi \times Vf \times Dp^2) \text{ Expression (4)}.$$

**7.** Procédé de production selon la revendication 5 ou la revendication 6,
dans lequel dans un cas où le nombre de fois Nb de saignée de la suspension cellulaire par jour est de un ou plus, un volume, qui est exprimé dans une unité de L, de la suspension cellulaire dans le récipient de culture est désigné par Vc, une valeur cible, qui est exprimée dans une unité de particules/mL, de Nd est désignée par Nd', et une densité de nombre, qui est exprimée dans une unité de particules/mL, de particules fines ayant une taille de particules de 8 Dp à 30 Dp autres que des cellules vivantes dans la suspension cellulaire avant n-ième saignée (n est un nombre naturel quelconque qui est Nb ou moins) est désignée par Ndn, la quantité Vbn, qui est exprimée dans une unité de L, de la n-ième saignée satisfait Expression (3) et Expression (5),

$$Vbn = Vc \times (Ndn - Nd')/Ndn \text{ Expression (3)}$$

$$2 \times 10^7 \text{ particules/mL} \leq Nd' \leq 2 \times 10^9 \text{ particules/mL Expression (5)}.$$

**8.** Procédé de production selon l'une quelconque des revendications 1 à 7,

dans lequel l'étape de culture d'une cellule qui produit un produit inclut l'agitation de la suspension cellulaire dans le récipient de culture avec une pale d'agitation, et
dans un cas où un coefficient de puissance de la pale d'agitation est désigné par Np, un diamètre de pale, qui est exprimé dans une unité de m, de la pale d'agitation est désigné par Di, une vitesse de rotation, qui est exprimée dans une unité de s$^{-1}$, de la pale d'agitation est désignée par Ro, une densité, qui est exprimée dans une unité de kg/m$^3$, de la suspension cellulaire est désignée par $\rho$, et un volume, qui est exprimé dans une unité de L, de la suspension cellulaire dans le récipient de culture est désigné par Vc, l'agitation est effectuée avec une puissance d'agitation Pv, qui est définie par Expression (6) et exprimée dans une unité de W/m$^3$, de la pale d'agitation dans une plage de 10 W/m³ à 150 W/m³, et dans un cas où une hauteur de niveau de liquide, qui est exprimée dans une unité de m, de la suspension cellulaire sur la base du fond du récipient de culture est désignée par Hc, une hauteur Hb, qui est exprimée dans une unité de m, d'une position d'extraction de saignée est de 1/2 × Hc ou moins,

$$Pv = Np \times \rho \times Ro^3 \times Di^5/(Vc \times 10^{-3}) \text{ Expression (6)}.$$

9. Procédé de production selon l'une quelconque des revendications 1 à 8,

dans lequel l'étape de culture d'une cellule qui produit un produit inclut le passage d'un gaz à travers la suspension cellulaire avec un diffuseur, et
dans un cas où une tension interfaciale, qui est exprimée dans une unité de N/m, de la suspension cellulaire est désignée par σ, un diamètre de pore, qui est exprimé dans une unité de μm, du diffuseur est désigné par Ds, un volume, qui est exprimé dans une unité de mL, d'une bulle de gaz unique générée à partir du diffuseur est désigné par Vg, un débit de passage de gaz, qui est exprimé dans une unité de mL/min, du diffuseur est désigné par Q, un volume, qui est exprimé dans une unité de L, de la suspension cellulaire dans le récipient de culture est désigné par Vc, une densité, qui est exprimée dans une unité de kg/m³, de la suspension cellulaire est désignée par ρ, et une accélération gravitationnelle exprimée dans une unité de m/s² est désignée par g, le nombre Ng, qui est défini par Expression (7) et Expression (8) et exprimé dans une unité de s⁻¹·L⁻¹, de gaz traversants par unité de capacité et par unité de temps satisfait Expression (9),

$$Ng = Q/(Vg \times Vc \times 60) \text{ Expression (7)}$$

$$Vg = \pi \times Ds \times \sigma/(\rho \times g) \text{ Expression (8)}$$

$$100 \text{ s}^{-1}\cdot\text{L}^{-1} \leq Ng \leq 5000 \text{ s}^{-1}\cdot\text{L}^{-1} \text{ Expression (9)}.$$

10. Procédé de production selon la revendication 9,
dans lequel la tension interfaciale σ de la suspension cellulaire et le diamètre de pore Ds du diffuseur satisfont respectivement Expression (10) et Expression (11),

$$10 \text{ mN/m} \leq \sigma \leq 100 \text{ mN/m} \text{ Expression (10)}$$

$$0,1 \text{ μm} \leq Ds \leq 200 \text{ μm} \text{ Expression (11)}.$$

11. Procédé de production selon l'une quelconque des revendications 1 à 10,
dans lequel la cellule est une cellule CHO.

12. Procédé de production selon l'une quelconque des revendications 1 à 11,

dans lequel le diamètre de pore Dp de la membrane de séparation est de 0,1 μm à 1 μm, et
pendant une période de culture après que la concentration en cellules vivantes a atteint une concentration maximale en cellules vivantes ou une concentration en cellules vivantes définie, Nd satisfait Expression (12) à tout moment,

$$2 \times 10^7 \leq Nd \leq 2 \times 10^9 \text{ Expression (12)}.$$

13. Procédé de production selon l'une quelconque des revendications 1 à 12,
dans lequel un taux de perméation du produit à travers la membrane de séparation, qui est calculé comme une proportion d'une concentration du produit contenu dans le liquide perméé à une concentration du produit contenu dans la suspension cellulaire, est de 55 % ou plus.

FIG. 1

100

## FIG. 2

## FIG. 3

EP 3 770 266 B1

## FIG. 4

## FIG. 5

33

FIG. 6

FIG. 7

# FIG. 8

# FIG. 9

EP 3 770 266 B1

FIG. 10

EP 3 770 266 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2016054686 A
- JP 2009045019 A
- JP 2007525984 A
- WO 2018159847 A1

**Non-patent literature cited in the description**

- **DALM MARCELLA C.F. et al.** Effect of feed and bleed rate on hybridoma cells in an acoustic perfusion bioreactor: Part I. Cell density, viability, and cell-cycle distribution. *BIOTECHNOLOGY AND BIOENGINEERING*, 05 December 2004, vol. 88 (5)

- **SAMANTHA WANG et al.** Shear contributions to cell culture performance and product recovery in ATF and TFF perfusion systems. *JOURNAL OF BIOTECHNOLOGY*, 01 March 2017, vol. 246